# EUROPEAN PATENT APPLICATION

(11) **EP 3 878 447 A1**
(43) Date of publication of application: **15.09.2021**
(21) Application number: 20162433.5
(22) Date of filing: 11.03.2020
(51) Int. Cl.: A61K 31/343, A61P 9/00, A61P 9/12, A61P 27/02, A61P 27/06, A61P 43/00

(54) **BETA ADRENERGIC RECEPTOR ANTAGONISTS**

(71) Applicant: InterAx Biotech AG, 5234 Villigen (CH)
(72) Inventor: Waldhoer, Maria, 8008 Zürich (CH); Zenone, Luca, 8046 Zürich (CH); Wilhelm, Florian, 5023 Biberstein (CH); Zimmermann, Mirjam, 4055 Basel (CH); Ostermaier, Martin Konrad, 79761 Waldshut-Tiengen (CH); Scharf, Magdalena Martina, 35037 Marburg (DE); Kolb, Peter, 35039 Marburg (DE)
(74) Representative: Latscha Schöllhorn Partner AG

(57) **Abstract**

The present invention relates to a β-adrenergic receptor antagonist and provides compositions and pharmaceutical compositions for the treatment, amelioration and / or prevention of disease.

## Description

The present invention relates to a β-adrenergic receptor antagonist and provides compositions and pharmaceutical compositions for the treatment, amelioration and/or prevention of disease.

### RELATED ART

G protein-coupled receptors (GPCRs) are involved in a plethora of physiological and pathophysiological processes in the human body and are targeted by approximately one third of currently marketed drugs (Hauser, Attwood, Rask-Andersen, Schiöth, & Gloriam, 2017; Salon, Lodowski, & Palczewski, 2011). One of the best-studied members of this large protein family are the adrenergic receptors. There are two main groups of adrenergic receptors, α and β. α-adrenergic receptors are divided into α1 and α2 receptors and β-adrenergic receptors are divided into β1, β2 and β3 receptors, β1-adrenergic receptors (B1AR) are typically involved in mediating cardiac acceleration and fatty acid mobilization, while β2-adrenergic receptors (B2AR) are typically involved in mediating smooth muscle relaxation.

Several agonists of these receptors are in clinical use such as for asthma medication. Antagonists of the B1AR/B2AR (= beta-blockers) have widely been used in the treatment of cardiovascular diseases (do Vale, Ceron, Gonzaga, Simplicio, & Padovan, Curr Hypertens Rev, 2019, 15(1): 22-31).

In the wake of the first X-ray structure of the B2AR (Cherezov et al., Science, 2007, Nov 23, 318(5854); Rosenbaum et al., Nature, 2007, 450), several computational docking studies reported new chemical entities (NCEs) for this receptor (Kolb et al., PNAS, 2009, April 21, 106 (16); Sabio, Jones, & Topiol, Bioorg Med Chem Lett, 2008, Oct 15: 18(20)). One of the ligands emerging from this screen targeting PDB ID 2RH1 was described by Kolb et al. (Kolb et al., PNAS, 2009, April 21, 106 (16) see termed compound 1 in Kolb et al.), which was later crystallized in complex with the B2AR leading to the corresponding crystal structure (PDB ID 3NY9; Wacker et al., 2010)).

Recently, the beta-blocker propranolol has been reported to be effective in the treatment of infantile hemangioma (Tsai, Liu, & Yeung, Medicine (Baltimore), 2019 Jan, 98(4): e14078). However, concerns about the effects of propranolol on the central nervous system (CNS) in infantile hemangioma patients have been raised (Thai, Wang, Chang, & Brown, J Clinc Med, 2019 Feb 22, 8(2)). Hence the quest for drug candidates acting at the β-adrenergic receptor, in particular at B1AR and/or B2AR is ongoing, despite its history of more than seven decades as a drug target.

### SUMMARY OF THE INVENTION

We have surprisingly found that compounds of formula (I) of the present invention are beta-adrenergic receptor antagonists, in particular beta-1 and beta-2-adrenergic receptor antagonists, based on computational docking and *in vitro* activity determination.

The computational docking focused on so far unexploited ligand-receptor interactions leading to unusual binding modes. The classical pharmacophore for the B2AR, as exemplified by adrenaline, consists of a protonable amine that interacts with Asp113^{3.32}, a hydroxyl group in β-position, and an aromatic moiety in 4-5 Å distance from the nitrogen. At the aromatic end, ligands can interact with the serines in helix 5, but this seems neither to be a requirement for affinity nor agonistic efficacy. The present invention thus focused additionally on ligand interactions with other amino acids in and around the orthosteric binding pocket for possible stabilization of unusual receptor conformations and feature interactions that have not yet been described for B2AR ligands, such as the interaction with extracellular loop 2 (ECL2). ECL2 is in proximity to the orthosteric binding pocket and interactions with it might represent a novel binding mode for ligands (Warne, Edwards, Leslie, & Tate, Structure, 2012, May 9, 20-540(5-7)).

The calculations were performed on two B2AR conformations of the receptor representing inactive states in complex with two different inverse agonists, i.e. in complex with carazolol (PDB 2RH1) (Cherezov et al., Science, 2007, Nov 23, 318(5854)) and in complex with a compound described by Kolb et al. (PDB ID 3NY9) (Kolb et al., PNAS, 2009, April 21, 106 (16), named compound 1 in Kolb et al.), respectively. The selection of these structures was based on the hypothesis that antagonists for the B2AR should be enriched in docking calculations to an inactive receptor conformation.

The compounds of formula (I) of the present invention were further tested *in vitro* to determine and confirm their binding affinities towards the B2AR in human embryonic kidney cells overexpressing a SNAP-tagged version of the B2AR (= SNAP-B2AR HEK).

Therefore, in an aspect, the present invention provides a compound of formula (I) or a pharmaceutically acceptable salt, solvate or hydrate thereof, 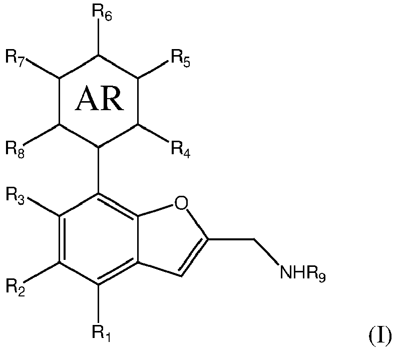 wherein
ring AR is phenyl or a 6-membered heteroaryl containing one or two heteroatoms selected from O, S and N;
R1, R2 and R3 are independently H, C₁-C₂alkyl, halogen, OH, O-C₁-C₂alkyl or NH₂;
R4, R5, R6, R7 and R8 are independently H, C₁-C₆alkyl, halogen, OH, OC₁-C₆alkyl, C₁-C₆alkyl-OH, N(R10)R11, C(O)OR12, C(O)N(R13)R14 or C₁-C₄alkyl-O-C₁-C₄alkyl;
R9 is H, C₁-C₃alkyl, halogen, C₁-C₃alkyl-OH or C(O)OR12; and wherein said
R10, R11, R12, R13 and R14 are independently H or C₁-C₃alkyl;
for use as a beta adrenergic receptor antagonist.

In another aspect, the invention relates to a compound of formula (I) or a pharmaceutically acceptable salt, solvate or hydrate thereof, as described herein, for use as a medicament.

In another aspect, the invention relates to a compound of formula (I) or a pharmaceutically acceptable salt, solvate or hydrate thereof as described herein, for use in treating a beta-1-adrenergic receptor related disease.

In another aspect, the invention relates to a compound of formula (I) or a pharmaceutically acceptable salt, solvate or hydrate thereof as described herein, for use in treating a beta-2-adrenergic receptor related disease.

In another aspect, the invention relates to a compound of formula (I) or a pharmaceutically acceptable salt, solvate or hydrate thereof as described herein for use in lowering blood pressure.

In another aspect, the invention relates to a method of treating a beta-adrenergic receptor related disease in a subject in need thereof, comprising administering to the subject an effective amount of a compound of formula (I) or a pharmaceutically acceptable salt, solvate or hydrate thereof as described herein.

In another aspect, the invention relates to a method of treating a beta-1-adrenergic receptor related disease in a subject in need thereof, comprising administering to the subject an effective amount of a compound of formula (I) or a pharmaceutically acceptable salt, solvate or hydrate thereof as described herein.

In another aspect, the invention relates to a method of treating a beta-2-adrenergic receptor related disease in a subject in need thereof, comprising administering to the subject an effective amount of a compound of formula (I) or a pharmaceutically acceptable salt, solvate or hydrate thereof as described herein.

In another aspect, the invention relates to a method of treating a cardiovascular disease, infantile hemangioma, glaucoma or disease related to the outer retina comprising administering an effective amount of a compound of formula (I) or a pharmaceutically acceptable salt, solvate or hydrate thereof as described herein to a subject in need thereof.

In another aspect, the invention relates to a method of selectively inhibiting a beta-adrenergic receptor in a cell, the method comprising: contacting a cell comprising a beta-adrenergic receptor with an effective amount of a compound of formula (I) or a pharmaceutically acceptable salt, solvate or hydrate thereof as described herein, to selectively inhibit the beta-adrenergic receptor in the cell.

These and further aspects and preferred embodiments thereof are also additionally defined below in the detailed description and in the claims.

### DESCRIPTION OF FIGURES

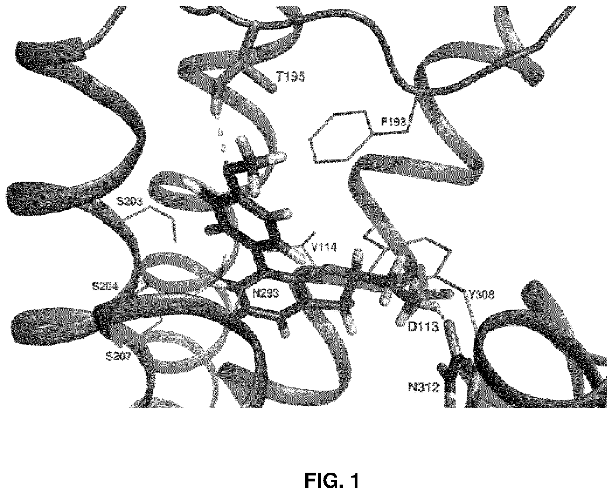

**Figure 1****:** Docked pose of Compound **1** in the orthosteric binding pocket of the B2AR as predicted by the docking calculation to the inactive conformation structure 3NY9. The molecule forms hydrogen bonds with residues D113^{3.32}, N312^{7.39} and T195 in ECL2.

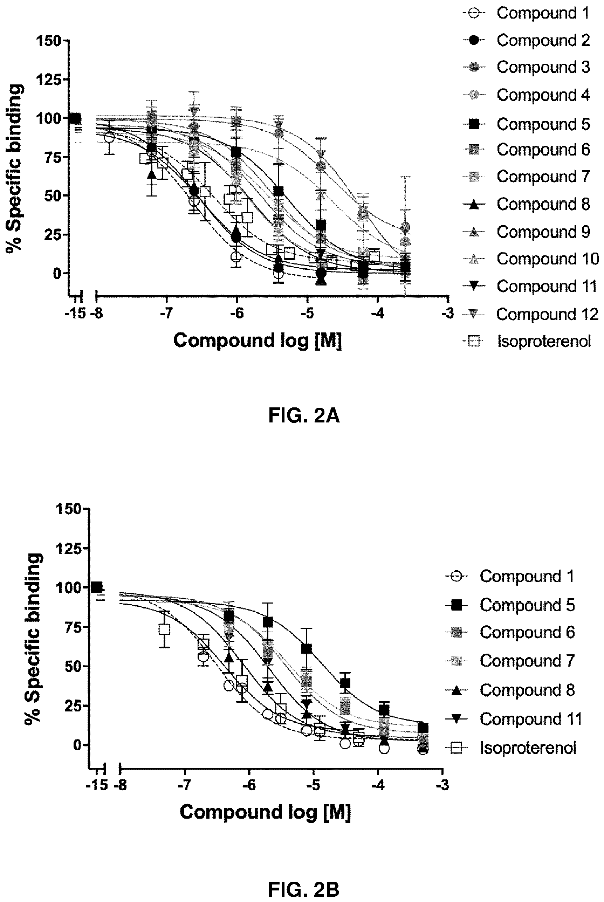

**Figure 2A****.** Heterologuous HTRF competition binding experiments with Propranolol-green on SNAP-B2AR HEK membranes. Heterologuous competition binding experiments were performed on SNAP-B2AR HEK membranes in the presence of 50 nM Propranolol-green and compounds of formula (I) in the concentrations indicated. Non-specific binding was determined in the presence of 10 µM ICI 118551. Specific binding in the absence of compounds of formula (I) was set to 100%. Isoproterenol was used as reference compound. Data represent mean ± SD from three to five independent experiments carried out in duplicates.
**Figure 2B****.** Heterologuous radioligand competition binding experiments with [³H]-DHA on SNAP-B2AR HEK membranes. The binding reaction was carried out in 200 µl containing membranes (5 µg of protein), WGA PVT SPA beads (500 µg), [³H]-DHA (final concentration 1 nM), and increasing concentrations of compounds of formula (I) for 120 min. Non-specific binding was determined in the presence of 1 µM ICI 118551. Specific binding in the absence of compounds of formula (I) was set to 100%. Data represent mean ± SD from three independent experiments carried out in duplicates.

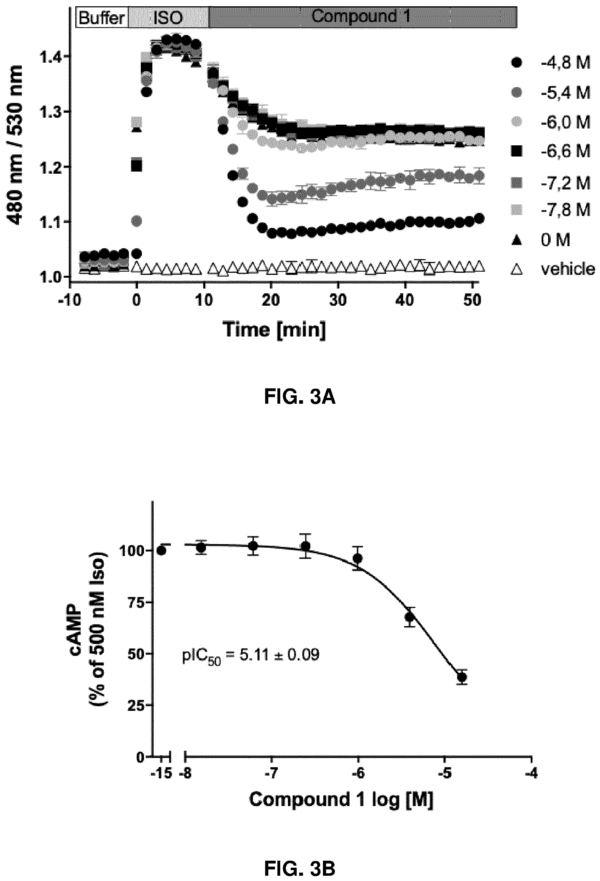

**Figure 3A****.** Real time traces of whole cell cAMP inhibition of Compound **1** in SNAP-B2AR-EPAC HEK cells. SNAP-B2AR-EPAC HEK cells were stimulated with 500 nM Isoproterenol for 10 min, followed by addition of Compound **1** in the concentrations indicated for an additional 40 min. The EPAC FRET ratio (mCerulean/mCitrine) was plotted as a function of time. Data represent mean ± SD of one representative out of five individual experiments carried out in duplicates.
**Figure 3B****.** Concentration-response curve of Compound **1** cAMP inhibition in SNAP-B2AR-EPAC HEK cells. The EPAC FRET ratio (mCerulean/mCitrine) was normalized to 500 nM Isoproterenol without inhibition (= 100%) and the area under the curve (AUC) was plotted as a function of Compound **1** concentrations. Data represent mean ± SD of one representative out of five individual experiments carried out in duplicates.

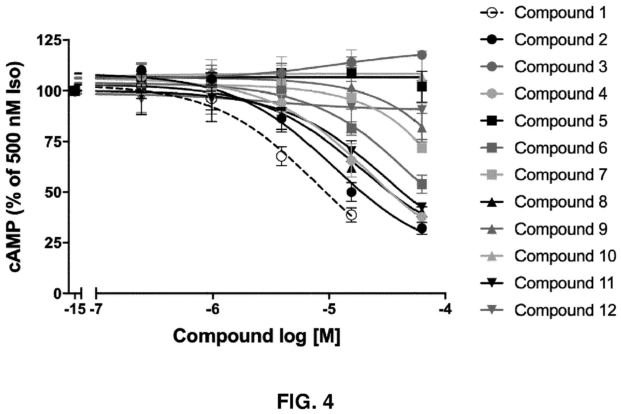

**Figure 4****.** Inhibition of whole cell cAMP in SNAP-B2AR-EPAC HEK cells by compounds of formula (I). SNAP-B2AR-EPAC HEK cells were stimulated with 500 nM Isoproterenol for 10 min, followed by addition of compounds of formula (I) in the concentrations indicated for an additional 40 min. The EPAC FRET ratio (mCerulean/mCitrine) was normalized to 500 nM Isoproterenol without inhibition (= 100%) and the area under the curve (AUC) was plotted as a function of the concentrations of compounds of formula (I). Data represent mean ± SD for three to five independent experiments carried out in duplicates.

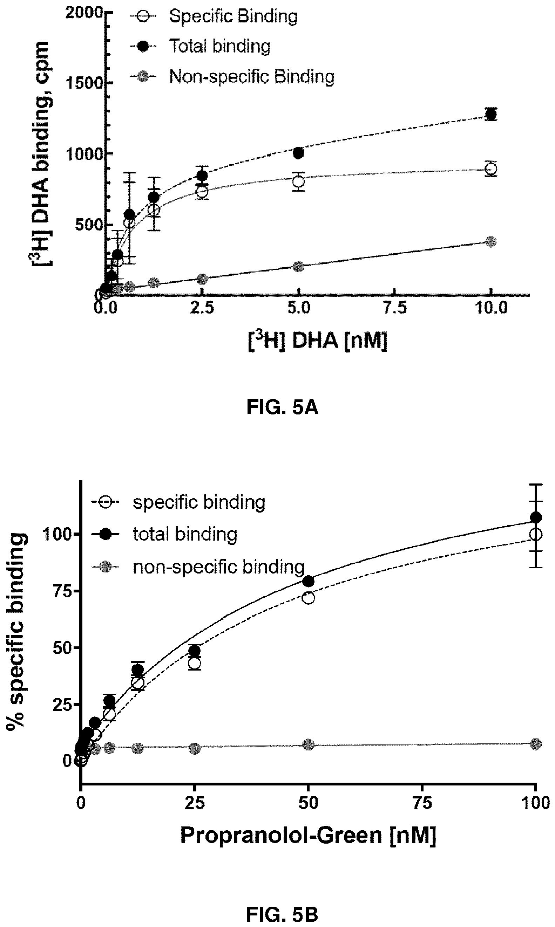

**Figure 5A****.** Saturation binding curve of Propranolol-Green in SNAP-B2AR HEK membranes. The binding reaction was carried out in 100 µl containing membranes (2 µg of protein) and increasing concentrations of Propranolol-green (ranging from 0.1 nM to 100 nM) for 120 min. Non-specific binding was determined in the presence of 10 µM ICI 118551. Data show mean ± SD of one representative out of two independent experiments measured in duplicates.
**Figure 5B****.** Saturation binding curve of [³H]-DHA in SNAP-B2AR HEK membranes. The binding reaction was carried out in 200 µl containing membranes (5 µg of protein), WGA PVT SPA beads (500 µg) and increasing concentrations of [³H]-DHA (ranging from 0.08 pM to 80 nM) for 120 min. Non-specific binding was determined in the presence of 1 µM ICI 118551. Data show mean ± SD of three independent experiments measured in duplicates.

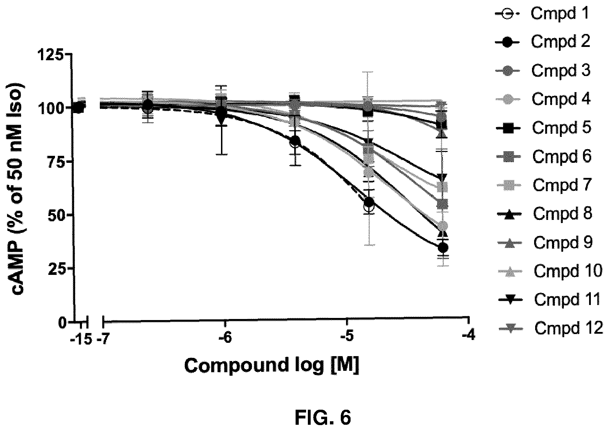

**Figure 6****.** Inhibition of whole cell cAMP in SNAP-B1AR-EPAC HEK cells. SNAP-B1AR-EPAC HEK cells were stimulated with 50 nM Isoproterenol for 10 min, followed by addition of compounds of formula (I) in the concentrations indicated for an additional 40 min. The EPAC FRET ratio (mCerulean/mCitrine) is normalized to 50 nM Isoproterenol without inhibition (= 100%) and the area under the curve (AUC) was plotted as a function of the concentrations of the compound of formula (I). Data were fitted to the equations described in Materials and Methods to calculate IC₅₀ values. Data represent mean ± SD from three to five independent experiments carried out in duplicates.

### DETAILED DESCRIPTION OF THE INVENTION

Unless defined otherwise, all technical and scientific terms used herein have the same meanings as commonly understood by one of ordinary skill in the art to which this invention belongs. The herein described and disclosed embodiments, preferred embodiments and very preferred embodiments should apply to all aspects and other embodiments, preferred embodiments and very preferred embodiments irrespective of whether it is specifically again referred to or its repetition is avoided for the sake of conciseness. The articles "a" and "an", as used herein, refer to one or to more than one (i.e., to at least one) of the grammatical object of the article. The term "or", as used herein, should be understood to mean "and/or", unless the context clearly indicates otherwise.

In an aspect, the present invention provides a compound of formula (I) or a pharmaceutically acceptable salt, solvate or hydrate thereof, 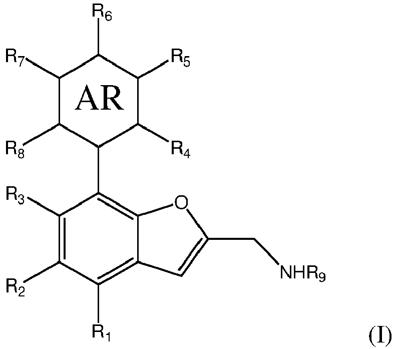 wherein
ring AR is phenyl or a 6-membered heteroaryl containing one or two heteroatoms selected from O, S and N;
R1, R2 and R3 are independently H, C₁-C₂alkyl, halogen, OH, O-C₁-C₂alkyl or NH₂;
R4, R5, R6, R7 and R8 are independently H, C₁-C₆alkyl, halogen, OH, OC₁-C₆alkyl, C₁-C₆alkyl-OH, N(R10)R11, C(O)OR12, C(O)N(R13)R14 or C₁-C₄alkyl-O-C₁-C₄alkyl;
R9 is H, C₁-C₃alkyl, halogen, C₁-C₃alkyl-OH or C(O)OR12; and wherein
R10, R11, R12, R13 and R14 are independently H or C₁-C₃alkyl.

In another aspect, the present invention provides a compound of formula (I) or a pharmaceutically acceptable salt, solvate or hydrate thereof, 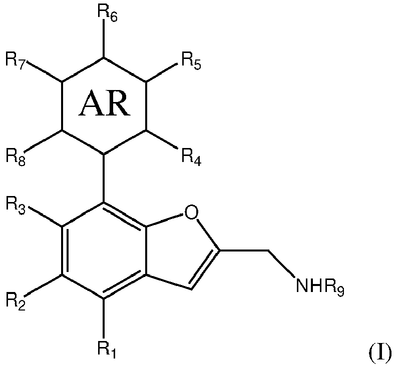 wherein
ring AR is phenyl or a 6-membered heteroaryl containing one or two heteroatoms selected from O, S and N;
R1, R2 and R3 are independently H, C₁-C₂alkyl, halogen, OH, O-C₁-C₂alkyl or NH₂;
R4, R5, R6, R7 and R8 are independently H, C₁-C₆alkyl, halogen, OH, OC₁-C₆alkyl, C₁-C₆alkyl-OH, N(R10)R11, C(O)OR12, C(O)N(R13)R14 or C₁-C₄alkyl-O-C₁-C₄alkyl;
R9 is H, C₁-C₃alkyl, halogen, C₁-C₃alkyl-OH or C(O)OR12; and wherein said
R10, R11, R12, R13 and R14 are independently H or C₁-C₃alkyl;
for use as a beta adrenergic receptor antagonist.

In a preferred embodiment, said ring AR is phenyl, pyridine, pyrimidine or pyrazine.

In a further preferred embodiment, said ring AR is selected from any of the formula 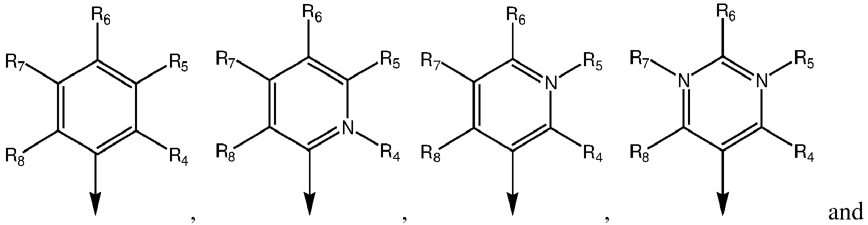 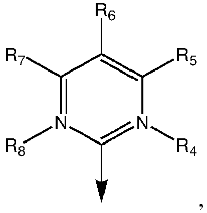 wherein the arrow denotes the bond in formula (I).

In a preferred embodiment, said ring AR is selected from any of the formula 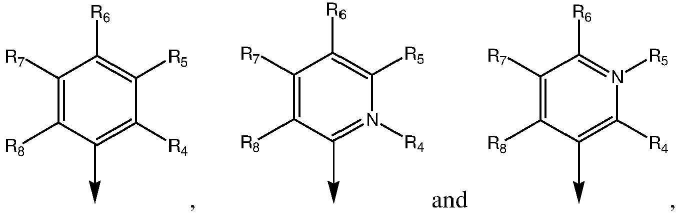 wherein the arrow denotes the bond in formula (I).

In a further preferred embodiment, said ring AR is 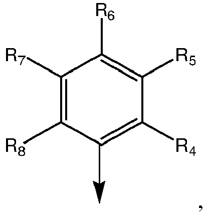 wherein the arrow denotes the bond in formula (I).

In a further preferred embodiment, said ring AR is 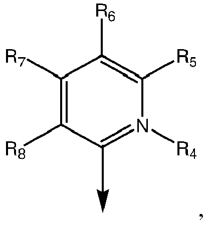 wherein the arrow denotes the bond in formula (I).

In a further preferred embodiment, said ring AR is 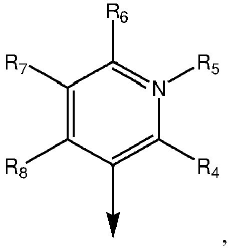 wherein the arrow denotes the bond in formula (I).

In a further preferred embodiment, at least one of said R1, R2 and R3 are H. In a further preferred embodiment, at least two of said R1, R2 and R3 are H. In a further preferred embodiment, R1 is H. In a further preferred embodiment, R1 is H, and R2 and R3 are independently H, C₁-C₂alkyl, halogen, OH, O-C₁-C₂alkyl or NH₂. In a further preferred embodiment, R1 is H, and R2 and R3 are independently H, halogen, OH, O-C₁-C₂alkyl or NH₂. In a further preferred embodiment, R1 is H, and R2 and R3 are independently H, halogen, OH, O-C₁-C₂alkyl or NH₂. In a further preferred embodiment, R1 is H, and R2 and R3 are independently H, F, Cl, OH, O-CH₃ or NH₂.

In a further preferred embodiment, said R9 is H, C₁-C₃alkyl, C₁-C₃alkyl-OH or C(O)OR12. In a further preferred embodiment, said R9 is H or C₁-C₃alkyl. In a further very preferred embodiment, said R9 is H or C₁-C₂alkyl. In a further very preferred embodiment, said R9 is H. In a further very preferred embodiment, said R9 is methyl. In a further very preferred embodiment, said R9 is ethyl.

In another embodiment, said R1 is H, and R2 and R3 are independently H, F, Cl or OH.

In a further preferred embodiment, R4, R5, R7 and R8 are independently H, C₁-C₂alkyl, halogen, OH, OC₁-C₂alkyl, C₁-C₂alkyl-OH, N(R10)R11; wherein said R10 and R11 are independently H or C₁-C₂alkyl.

In a further preferred embodiment, R4, R5, R7 and R8 are independently H, methyl, halogen, OH, OCH₃, CH₂-OH, N(R10)R11; wherein said R10 and R11 are independently H or methyl.

In a further preferred embodiment, R6 is H, OH, OC₁-C₆alkyl, C₁-C₆alkyl-OH, N(R10)R11, C(O)OR12, C(O)N(R13)R14 or C₁-C₆alkyl-O-C₁-C₃alkyl; wherein said R10, R11, R12, R13 and R14 are independently H or C₁-C₃alkyl.

In a further preferred embodiment, R6 is H, OH, OC₁-C₃alkyl, C₁-C₃alkyl-OH, N(R10)R11, C(O)OR12, C(O)N(R13)R14 or C₁-C₃alkyl-O-C₁-C₃alkyl; wherein said R10, R11, R12, R13 and R14 are independently H or C₁-C₃alkyl.

In a further preferred embodiment, R4, R5, R7 and R8 are independently H, C₁-C₂alkyl, halogen, OH, OC₁-C₂alkyl, C₁-C₂alkyl-OH, N(R10)R11; wherein said R10 and R11 are independently H or C₁-C₂alkyl, and wherein R6 is H, OH, OC₁-C₆alkyl, C₁-C₆alkyl-OH, N(R10)R11, C(O)OR12, C(O)N(R13)R14 or C₁-C₆alkyl-O-C₁-C₃alkyl, and wherein preferably R6 is H, OH, OC₁-C₃alkyl, C₁-C₃alkyl-OH, N(R10)R11, C(O)OR12, C(O)N(R13)R14 or C₁-C₃alkyl-O-C₁-C₃alkyl; wherein said R10, R11, R12, R13 and R14 are independently H or C₁-C₃alkyl.

In a further preferred embodiment, R4, R5, R7 and R8 are independently H, methyl, halogen, OH, OCH₃, CH₂-OH, N(R10)R11; wherein said R10 and R11 are independently H or methyl, and wherein R6 is H, OH, OC₁-C₃alkyl, C₁-C₃alkyl-OH, N(R10)R11, C(O)OR12, C(O)N(R13)R14 or C₁-C₃alkyl-O-C₁-C₃alkyl; wherein said R10, R11, R12, R13 and R14 are independently H or C₁-C₃alkyl.

In a further preferred embodiment, at least one of said R4, R5, R6, R7 and R8 are H. In a further preferred embodiment, at least two of said R4, R5, R6, R7 and R8 are H. In a further preferred embodiment, R4 and R8 is H.

In a further preferred embodiment, at least two of said R4, R5, R6, R7 and R8 are H, and wherein the other of said R4, R5, R6, R7 and R8 are independently H, C₁-C₃alkyl, halogen, OH, OC₁-C₃alkyl, C₁-C₃alkyl-OH, N(R10)R11, C(O)OR12, C(O)N(R13)R14 or C₁-C₃alkyl-O-C₁-C₃alkyl; wherein said R10, R11, R12, R13 and R14 are independently H or C₁-C₃alkyl. In a further preferred embodiment, at least two of said R4, R5, R6, R7 and R8 are H, and wherein the other of said R4, R5, R6, R7 and R8 are independently H, F, Cl, OH, OC₁-C₂alkyl, C₁-C₂alkyl-OH, N(R10)R11, C(O)OR12, C(O)N(R13)R14 or C₁-C₂alkyl-O-C₁-C₂alkyl; wherein said R10, R11, R12, R13 and R14 are independently H or C₁-C₂alkyl.

In a further preferred embodiment, R4 and R8 are H, and R5, R6 and R7 are independently H, C₁-C₃alkyl, halogen, OH, OC₁-C₃alkyl, C₁-C₃alkyl-OH, N(R10)R11, C(O)OR12, C(O)N(R13)R14 or C₁-C₃alkyl-O-C₁-C₃alkyl; wherein said R10, R11, R12, R13 and R14 are independently H or C₁-C₃alkyl. In a further preferred embodiment, R4 and R8 is H, and R5, R6 and R7 are independently H, F, Cl, OH, OC₁-C₂alkyl, C₁-C₂alkyl-OH, N(R10)R11, C(O)OR12, C(O)N(R13)R14 or C₁-C₂alkyl-O-C₁-C₂alkyl; wherein said R10, R11, R12, R13 and R14 are independently H or C₁-C₂alkyl. In a further preferred embodiment, R4 and R8 is H, and R5, R6 and R7 are independently H, F, Cl, OH, OC₁-C₂alkyl, C₁-C₂alkyl-OH, N(R10)R11, C(O)OR12, C(O)N(R13)R14; wherein said R10, R11, R12, R13 and R14 are independently H or C₁-C₂alkyl.

In a further preferred embodiment, R4 and R8 are H, and R5, R6 and R7 are independently H, C₁-C₃alkyl, halogen, OH, OC₁-C₃alkyl, C₁-C₃alkyl-OH, N(R10)R11, C(O)OR12, C(O)N(R13)R14 or C₁-C₃alkyl-O-C₁-C₃alkyl; wherein said R10, R11, R12, R13 and R14 are independently H or C₁-C₃alkyl. In a further preferred embodiment, R4 and R8 is H, and R5, R6 and R7 are independently H, F, Cl, OH, OC₁-C₂alkyl, C₁-C₂alkyl-OH, N(R10)R11, C(O)OR12, C(O)N(R13)R14 or C₁-C₂alkyl-O-C₁-C₂alkyl; wherein said R10, R11, R12, R13 and R14 are independently H or C₁-C₂alkyl. In a further preferred embodiment, R4 and R8 is H, and R5, R6 and R7 are independently H, F, Cl, OH, OC₁-C₂alkyl, C₁-C₂alkyl-OH, N(R10)R11, C(O)OR12, C(O)N(R13)R14; wherein said R10, R11, R12, R13 and R14 are independently H or C₁-C₂alkyl.

In a preferred embodiment, said R4 and R8 are H, and R5, R6 and R7 are independently H, F, Cl, OH, OCH₃, CH₂-OH, N(R10)R11, C(O)OR12, C(O)N(R13)R14; wherein said R10, R11, R12, R13 and R14 are independently H or methyl.

In a further preferred embodiment, R4 and R8 are H; R5 and R7 are independently H, F, Cl, OH, OC₁-C₂alkyl, C₁-C₂alkyl-OH, N(R10)R11; and R6 is H, F, Cl, OH, OC₁-C₃alkyl, C₁-C₃alkyl-OH, N(R10)R11, C(O)OR12, C(O)N(R13)R14; wherein said R10, R11, R12, R13 and R14 are independently H or C₁-C₃alkyl. In a further preferred embodiment, R4 and R8 are H; R5 and R7 are independently H, F, Cl, OH, OC₁-C₂alkyl, C₁-C₂alkyl-OH N(R10)R11; and R6 is H, F, Cl, OH, OC₁-C₂alkyl, C₁-C₂alkyl-OH, N(R10)R11, C(O)OR12, C(O)N(R13)R14; wherein said R10, R11, R12, R13 and R14 are independently H or C₁-C₂alkyl. In a further preferred embodiment, R4 and R8 are H; R5 and R7 are independently H, F, Cl, OH, OCi-C₃alkyl, C₁-C₃alkyl-OH, N(R10)R11; and R6 is OH, OC₁-C₃alkyl, C₁-C₃alkyl-OH, N(R10)R11, C(O)OR12, C(O)N(R13)R14; wherein said R10, R11, R12, R13 and R14 are independently H or C₁-C₃alkyl. In a further preferred embodiment, R4 and R8 are H; R5 and R7 are independently H, F, Cl, OH, OC₁-C₂alkyl, C₁-C₂alkyl-OH, NH₂; and R6 is OH, OC₁-C₂alkyl, C₁-C₂alkyl-OH, N(R10)R11, C(O)OR12, C(O)N(R13)R14; wherein said R10, R11, R12, R13 and R14 are independently H or C₁-C₂alkyl.

In a further preferred embodiment, R4 and R8 are H; R5 and R7 are independently H, F, Cl, OH, OC₁-C₂alkyl, C₁-C₂alkyl-OH, NH₂, wherein one of said R5 and R7 is H; and R6 is H, F, Cl, OH, OC₁-C₃alkyl, C₁-C₃alkyl-OH, N(R10)R11, C(O)OR12, C(O)N(R13)R14; wherein said R10, R11, R12, R13 and R14 are independently H or C₁-C₃alkyl. In a further preferred embodiment, R4 and R8 are H; R5 and R7 are independently H, F, Cl, OH, OC₁-C₂alkyl, C₁-C₂alkyl-OH, NH₂, wherein one of said R5 and R7 is H; and R6 is H, F, Cl, OH, OC₁-C₂alkyl, C₁-C₂alkyl-OH, N(R10)R11, C(O)OR12, C(O)N(R13)R14; wherein said R10, R11, R12, R13 and R14 are independently H or C₁-C₂alkyl. In a further preferred embodiment, R4 and R8 are H; R5 and R7 are independently H, F, Cl, OH, OC₁-C₃alkyl, C₁-C₃alkyl-OH, NH₂, wherein one of said R5 and R7 is H; and R6 is OH, OC₁-C₃alkyl, C₁-C₃alkyl-OH, N(R10)R11, C(O)OR12, C(O)N(R13)R14; wherein said R10, R11, R12, R13 and R14 are independently H or C₁-C3alkyl. In a further preferred embodiment, R4 and R8 are H; R5 and R7 are independently H, F, Cl, OH, OC₁-C₂alkyl, C₁-C₂alkyl-OH, NH₂, wherein one of said R5 and R7 is H; and R6 is OH, OC₁-C₂alkyl, C₁-C₂alkyl-OH, N(R10)R11, C(O)OR12, C(O)N(R13)R14; wherein said R10, R11, R12, R13 and R14 are independently H or C₁-C₂alkyl. In a further preferred embodiment, R4 and R8 are H; R5 and R7 are independently H, F, Cl, OH, OCH₃, CH₂-OH, NH₂, wherein one of said R5 and R7 is H; and R6 is OH, OCH₃, CH₂-OH, N(R10)R11, C(O)OR12, C(O)N(R13)R14; wherein said R10, R11, R12, R13 and R14 are independently H or methyl.

In another embodiment said R4 and R8 are H; R5 and R7 are independently H, F, Cl, OH, OCH₃, CH₂-OH, NH₂; and R6 is OH, OCH₃, CH₂-OH, N(R10)R11, C(O)OR12, C(O)N(R13)R14; wherein said R10, R11, R12, R13 and R14 are independently H or methyl.

In a further preferred embodiment, R5 and R7 are independently H, F, Cl, OH, OCH₃, CH₂-OH, NH₂, wherein one of said R5 and R7 is H. In a further preferred embodiment, R5 and R7 are independently H, F, OH, OCH₃, NH₂, wherein one of said R5 and R7 is H. In a further preferred embodiment, R5 and R7 are independently H and F, wherein one of said R5 and R7 is H.

In a further preferred embodiment, R6 is OH, OC₁-C₂alkyl, C₁-C₂alkyl-OH, N(R10)R11, C(O)OR12, C(O)N(R13)R14; wherein said R10, R11, R12, R13 and R14 are independently H or C₁-C₂alkyl. In a further preferred embodiment, R6 is OH, OCH₃, CH₂-OH, N(R10)R11, C(O)OR12, C(O)N(R13)R14; wherein said R10, R11, R12, R13 and R14 are independently H or methyl. In a further preferred embodiment, R6 is OH, OCH₃, N(R10)R11, C(O)OR12, C(O)N(R13)R14; wherein said R10, R11, R12, R13 and R14 are independently H or methyl.

In a further preferred embodiment, R6 is OH, OC₁-C₂alkyl, C₁-C₂alkyl-OH, N(R10)R11, C(O)OR12, C(O)N(R13)R14. In another preferred embodiment, R6 is OH, OCH₃, CH₂-OH, N(R10)R11, C(O)OR12, C(O)N(R13)R14. In another preferred embodiment, R6 is OH, OCH₃, N(R10)R11, C(O)OR12, C(O)N(R13)R14. In a further preferred embodiment, R6 is OH, OCH₃, NH₂, C(O)OH, C(O)OCH₃, C(O)NH₂. In another preferred embodiment, R6 is OH or OCH3. In a further preferred embodiment, R6 is OH. In a further preferred embodiment, R6 is OCH3. In a further preferred embodiment, R6 is NH₂. In a further preferred embodiment, R6 is C(O)NH₂.

In another embodiment said R4 and R8 are H; R5 and R7 are independently H, F, OH, OCH₃, NH₂, wherein one of said R5 and R7 is H; and R6 is OH, OCH₃, N(R10)R11, C(O)OR12, C(O)N(R13)R14; wherein said R10, R11, R12, R13 and R14 are independently H or methyl.

In another embodiment said R4 and R8 are H; R5 and R7 are independently H and F, wherein one of said R5 and R7 is H; and R6 is OH, OCH₃, NH₂, C(O)OH, C(O)OCH₃, C(O)NH₂.

In another embodiment said compound is selected from

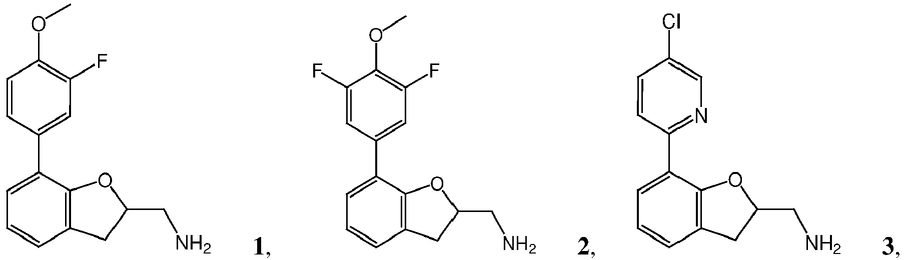

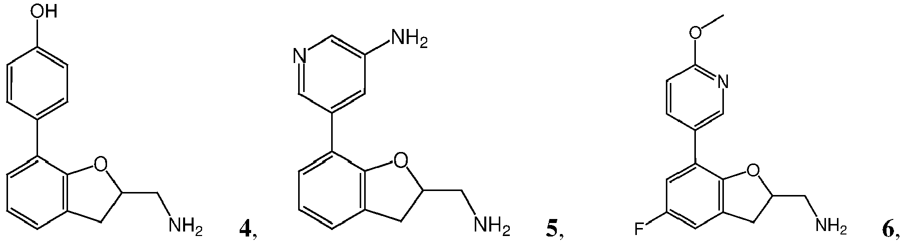

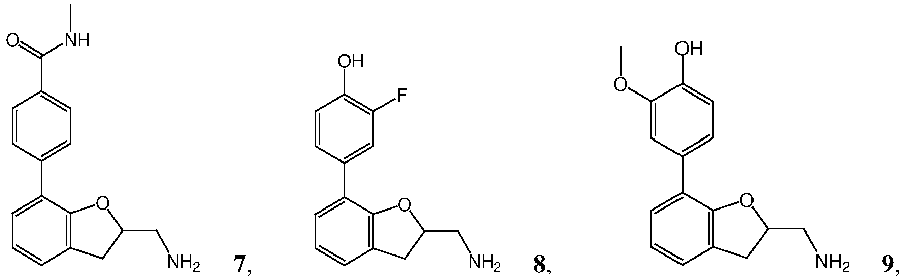

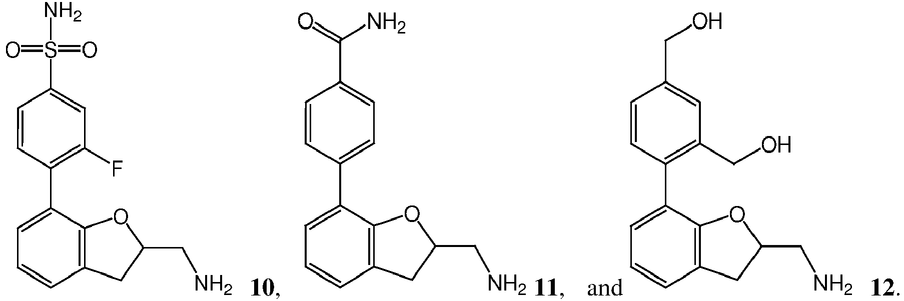

The term "alkyl", as used herein, refers to a straight or branched hydrocarbon chain radical consisting solely of carbon and hydrogen atoms, containing no unsaturation, having typically and preferably from one to three carbon atoms (*e.g.,* (C₁-₃alkyl), and which typically is attached to the rest of the molecule by a single bond. Whenever it appears herein, a numerical range such as "1 to 3" refers to each integer in the given range. For example, "1 to 3 carbon atoms" means that the alkyl group may consist of 1 carbon atom, 2 carbon atoms or 3 carbon atoms. Typical alkyl groups include methyl, ethyl, *n*-propyl, prop-2-yl.

Halogen is fluorine, chlorine, bromine, or iodine, preferably fluorine.

The term "heteroaryl" refers to an aromatic ring system containing one or two, preferably one, heteroatoms selected from nitrogen, oxygen and sulfur as ring members. Preferred examples include in particular pyridinyl, pyrimidinyl, pyrazinyl, pyridazinyl and thiophenyl. Thus, in a preferred embodiment, said 6-membered heteroaryl is selected from pyridinyl, pyrimidinyl, pyrazinyl, pyridazinyl and thiophenyl. In a very preferred embodiment said 6-membered heteroaryl is pyridinyl.

Certain compounds of formula (I) may contain one or two or more centers of chirality and such compounds may be provided as pure enantiomers or pure diastereoisomers as well as mixtures thereof in any ratio.

A "solvate" refers to an association or complex of one or more solvent molecules and a compound of formula (I) of the present invention. Examples of solvents that form solvates include, but are not limited to, water, isopropanol, ethanol, methanol, dimethyl sulfoxide (DMSO), ethyl acetate, acetic acid, and ethanolamine. The term "hydrate" refers to the complex where the solvent molecule is water.

The term "pharmaceutically acceptable salts" or "pharmaceutically acceptable salt" refers to inorganic and organic salts of a compound of the present invention. These salts can be prepared in situ during the final isolation and purification of a compound, or by separately reacting the compound with a suitable organic or inorganic acid and isolating the salt thus formed. Representative salts include the hydrobromide, hydrochloride, hydroiodide, sulfate, bisulfate, nitrate, acetate, trifluoroacetate, oxalate, besylate, palmitiate, pamoate, malonate, stearate, laurate, malate, borate, benzoate, lactate, phosphate, hexafluorophosphate, benzene sulfonate, tosylate, formate, citrate, maleate, fumarate, succinate, tartrate, naphthylate, mesylate, glucoheptonate, lactobionate, and laurylsulphonate salts, and similarly known acceptable acids.

As is known in the art, beta adrenergic receptor antagonists comprise a class of drug compounds that are typically used for management of cardiac arrhythmias, inhibition of secondary myocardial infarction, management of hypertension, and other indications. The present disclosure includes using any one or any combination of beta adrenergic antagonists disclosed herein, that are antagonists of any one or any combination of the three presently known types of beta receptors (β1, β2 and β3 receptors), or otherwise interfere with one or more of these receptors binding to their endogenous ligands, i.e., epinephrine and/or other stress hormones.

As used herein, the term "antagonist" means any molecule that blocks the ability of a given chemical to bind to its receptor, thereby preventing a biological response. The term antagonist can be used in a functional sense and is not intended to limit the invention to compounds having a particular mechanism of action. For example, the term "antagonist" includes, but is not limited to, molecules that function as competitive antagonists. A "competitive antagonist" is one which binds the receptor but does not trigger the biological activity of the receptor.

In other aspects, the present invention provides the compound of formula (I) for use according to the invention, wherein the beta adrenergic receptor antagonist is a beta-1-adrenergic receptor antagonist. In other aspects, the present invention provides the compound of formula (I) for use according to the invention, wherein the beta adrenergic receptor antagonist is a beta-2-adrenergic receptor antagonist.

Beta-1-adrenergic receptors are found primarily in the heart, blood vessels and adipose tissue. Beta-2-adrenergic receptors are found primarily in skeletal and smooth muscle, bone, cartilage, connective tissue, the intestines, lungs, bronchial glands and liver.

In another aspect, the present invention provides a compound of formula (I) or a pharmaceutically acceptable salt, solvate or hydrate thereof, 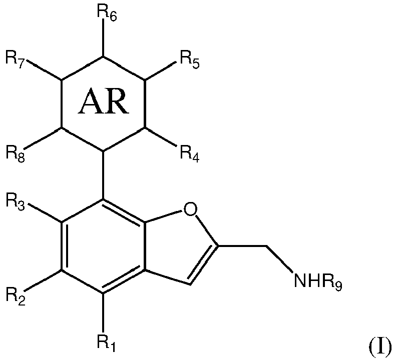 wherein
ring AR is phenyl or a 6-membered heteroaryl ring containing one or two heteroatoms selected from O, S and N;
R1, R2 and R3 are independently H, C₁-C₂alkyl, halogen, OH, O-C₁-C₂alkyl or NH₂;
R4, R5, R6, R7 and R8 are independently H, C₁-C₆alkyl, halogen, OH, OC₁-C₆alkyl, C₁-C₆alkyl-OH, N(R10)R11, C(O)OR12, C(O)N(R13)R14 or C₁-C₄alkyl-O-C₁-C₄alkyl;
R9 is H, C₁-C₃alkyl, halogen, C₁-C₃alkyl-OH or C(O)OR12; and wherein said
R10, R11, R12, R13 and R14 are independently H or C₁-C₃alkyl;
for use as a medicament.

In another aspect, the present invention provides a compound of formula (I), 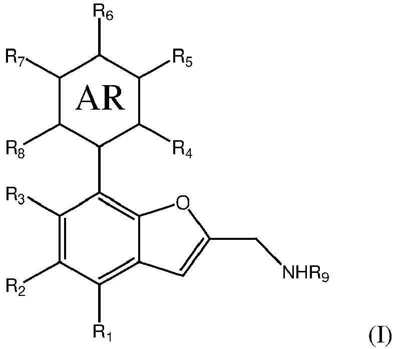 wherein
ring AR is phenyl or a 6-membered heteroaryl ring containing one or two heteroatoms selected from O, S and N;
R1, R2 and R3 are independently H, C₁-C₂alkyl, halogen, OH, O-C₁-C₂alkyl or NH₂;
R4, R5, R6, R7 and R8 are independently H, C₁-C₆alkyl, halogen, OH, OC₁-C₆alkyl, C₁-C₆alkyl-OH, N(R10)R11, C(O)OR12, C(O)N(R13)R14 or C₁-C₄alkyl-O-C₁-C₄alkyl;
R9 is H, C₁-C₃alkyl, halogen, C₁-C₃alkyl-OH or C(O)OR12; and wherein said
R10, R11, R12, R13 and R14 are independently H or C₁-C₃alkyl;
for use as a medicament.

In another aspect, the present invention provides a compound or a pharmaceutically acceptable salt of formula (I) or a pharmaceutically acceptable salt, solvate or hydrate thereof, 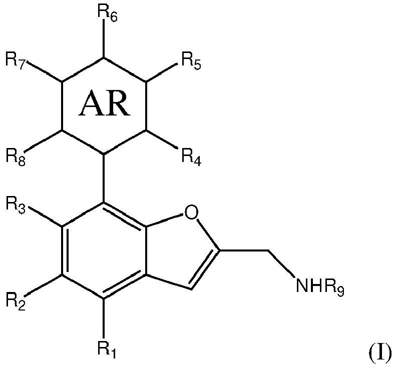 wherein
ring AR is phenyl or a 6-membered heteroaryl ring containing one or two heteroatoms selected from O, S and N;
R1, R2 and R3 are independently H, C₁-C₂alkyl, halogen, OH, O-C₁-C₂alkyl or NH₂;
R4, R5, R6, R7 and R8 are independently H, C₁-C₆alkyl, halogen, OH, OC₁-C₆alkyl, C₁-C₆alkyl-OH, N(R10)R11, C(O)OR12, C(O)N(R13)R14 or C₁-C₄alkyl-O-C₁-C₄alkyl;
R9 is H, C₁-C₃alkyl, halogen, C₁-C₃alkyl-OH or C(O)OR12; and wherein said
R10, R11, R12, R13 and R14 are independently H or C₁-C₃alkyl;
for use in treating a beta-2-adrenergic receptor related disease.

In another aspect, the present invention provides a compound of formula (I), 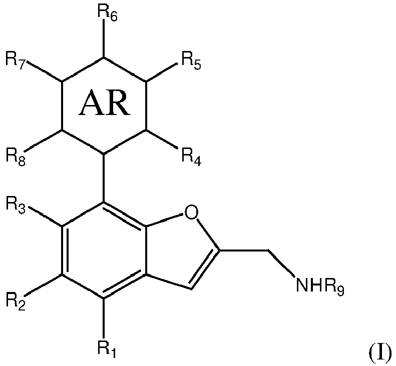 wherein
ring AR is phenyl or a 6-membered heteroaryl ring containing one or two heteroatoms selected from O, S and N;
R1, R2 and R3 are independently H, C₁-C₂alkyl, halogen, OH, O-C₁-C₂alkyl or NH₂;
R4, R5, R6, R7 and R8 are independently H, C₁-C₆alkyl, halogen, OH, OC₁-C₆alkyl, C₁-C₆alkyl-OH, N(R10)R11, C(O)OR12, C(O)N(R13)R14 or C₁-C₄alkyl-O-C₁-C₄alkyl;
R9 is H, C₁-C₃alkyl, halogen, C₁-C₃alkyl-OH or C(O)OR12; and wherein said
R10, R11, R12, R13 and R14 are independently H or C₁-C₃alkyl;
for use in treating a beta-adrenergic receptor related disease, preferably a beta-1-adrenergic receptor related disease or a beta-2-adrenergic receptor related disease, and further preferably a beta-2-adrenergic receptor related disease.

In a preferred embodiment the beta-1-adrenergic receptor related disease is a disease that is caused by a dysfunction of the beta-1-adrenegic receptor. The disease may be caused by an over-activation of the beta-1-adrenergic receptor or an inhibition of the beta-1-adrenergic receptor. The disease may be caused by a mutation in the beta-1-adrenergic receptor. In a preferred embodiment the beta-2-adrenergic receptor related disease is a disease that is caused by a dysfunction of the beta-2-adrenegic receptor. The disease may be caused by an over-activation of the beta-2-adrenergic receptor or an inhibition of the beta-2-adrenergic receptor. The disease may be caused by a mutation in the beta-2-adrenergic receptor.

In a preferred embodiment, the beta-1-adrenergic receptor related disease is a cardiovascular disease, hypertension, infantile hemangioma, glaucoma or disease related to the outer retina. In a preferred embodiment, the beta-2-adrenergic receptor related disease is a cardiovascular disease, hypertension, infantile hemangioma, glaucoma or disease related to the outer retina.

In a more preferred embodiment, the beta-1-adrenergic receptor related disease is a cardiovascular disease. In a more preferred embodiment, the beta-2-adrenergic receptor related disease is a cardiovascular disease. In a more preferred embodiment, the beta-1-adrenergic receptor related disease is hypertension. In a more preferred embodiment, the beta-2-adrenergic receptor related disease is hypertension. In an even more preferred embodiment, the cardiovascular disease is characterized by high blood pressure.

In a still more preferred embodiment, the beta-1-adrenergic receptor related disease is an infantile hemangioma. In a still more preferred embodiment, the beta-2-adrenergic receptor related disease is an infantile hemangioma. In a further preferred embodiment, said beta-1-adrenergic receptor related disease is glaucoma. In a further preferred embodiment, said beta-1-adrenergic receptor related disease is a disease related to the outer retina. In a further preferred embodiment, said beta-2-adrenergic receptor related disease is glaucoma. In a further preferred embodiment, said beta-2-adrenergic receptor related disease is a disease related to the outer retina.

In another aspect, the present invention provides a compound or a pharmaceutically acceptable salt of formula (I) or a pharmaceutically acceptable salt, solvate or hydrate thereof, 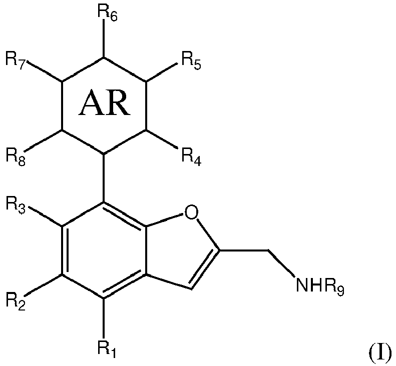 wherein
ring AR is phenyl or a 6-membered heteroaryl ring containing one or two heteroatoms selected from O, S and N;
R1, R2 and R3 are independently H, C₁-C₂alkyl, halogen, OH, O-C₁-C₂alkyl or NH₂;
R4, R5, R6, R7 and R8 are independently H, C₁-C₆alkyl, halogen, OH, OC₁-C₆alkyl, C₁-C₆alkyl-OH, N(R10)R11, C(O)OR12, C(O)N(R13)R14 or C₁-C₄alkyl-O-C₁-C₄alkyl;
R9 is H, C₁-C₃alkyl, halogen, C₁-C₃alkyl-OH or C(O)OR12; and wherein said
R10, R11, R12, R13 and R14 are independently H or C₁-C₃alkyl;
for use in treating a beta-1-adrenergic receptor related disease.

In a preferred embodiment the beta-1-adrenergic receptor related disease is a disease that is caused by a dysfunction of the beta-1-adrenegic receptor. The disease may be caused by an over-activation of the beta-1-adrenergic receptor or an inhibition of the beta-1-adrenergic receptor. The disease may be caused by a mutation in the beta-1-adrenergic receptor.

In a preferred embodiment, the beta-1-adrenergic receptor related disease is hypertension, angina pectoris, arrhythmias, coronary heart disease, myocardial infarction, glaucoma, ischemic heart disease, infectious and non-infectious heart disease, ischemic heart and non-ischemic heart disease, inflammatory heart disease, myocarditis, cardiac dilation, idiopathic cardiomyopathy, idiopathic dilated cardiomyopathy, immune cardiomyopathy, heart failure or depression.

The compounds and compositions of this invention can be used with other drugs to provide a combination therapy. The other drugs can be part of the same composition or can be provided as a separate composition for administration at the same time or at a different time.

As it is used herein, the terms "to treat", "treating" and "treatment" generally include the eradication, elimination, reversal, alleviation, modification or control of a disease in a subject. As it is used herein, the terms "prevention", "preventing", "preventive", "to prevent" and prophylaxis refer to the capability of a given substance to thwart, minimize or complicate the onset or development of a disease in a subject.

The term "subject" or "patient" in the context of the invention includes any animal, particularly vertebrate animals, preferably mammals, such as mice, rats, horses, pigs, rabbits, cats, sheep, dogs, cows, human beings, etc. In a preferred embodiment, the mammal is a pig or a human being. In another even more preferred embodiment, the mammal is a human being.

The medical indications for the use of the beta adrenergic receptor antagonists disclosed herein are: hypertension, coronary heart disease, myocardial infarction, heart failure and cardiac arrhythmias, hyperthyroidism, tremor, glaucoma, migraine, pheochromocytoma, anxiety disorder, portal hypertension, prevention of esophageal variceal hemorrhage, hemangioma, duming syndrome and Ehlers-Danlos syndrome.

The other medical indications for the use of the beta adrenergic antagonists disclosed herein are: oncology (metastasis, cancers of breast, leukemia, melanoma, ovarian, angiosarcoma, neuroblastoma, prostate, colorectal, pancreatic and others), anti-depressant, polydipsia in schizophrenics, polyneuropathy, PTSD and memory suppression.

According to the present invention, the medicament may be a medicament for treating infantile capillary hemangiomas. According to the present invention, the medicament may also be a medicament for treating other vascular tumors, for example those selected from the group consisting of hemangiomas (i.e. epithelioid hemangioma, sinusoidal hemangioma, spindle cell hemangioma), tufted angioma, hemangioendotheliomas (i.e. kaposiform hemangioendothelioma), hemangioma in the von Hippel-Lindau syndrome, angiofibroma and angiolipoma in Bourneville disease, pyogenic granuloma, angiosarcomas, for example Kaposi's sarcoma, expanding arteriovenous malformations and tumor-associated vascular proliferation (M. Wassef, Ann Chir Plast Esthet, 2006; 51:263-281).

The medicament may be in any form that can be administered to a human or an animal. Administration may be carried out directly, i.e. pure or substantially pure, or after mixing of the beta adrenergic receptor antagonist with a pharmaceutically acceptable carrier and/or medium. According to the present invention, the medicament may be a syrup or an injectable solution. According to the present invention, the medicament may be a medicament for oral administration selected from the group comprising a liquid formulation, an oral effervescent dosage form, an oral powder, a multiparticule system, an orodispersible dosage form.

For example, when the medicament is a medicament for oral administration, it may be in the form of a liquid formulation selected from the group comprising a solution, a syrup, a suspension, an emulsion and oral drops. When the medicament is in the form of an oral effervescent dosage form, it may be in a form selected from the group comprising tablets, granules, powders. When the medicament is the form of an oral powder or a multiparticulate system, it may be in a form selected from the group comprising beads, granules, mini tablets and micro granules. When the medicament is the form of an orodispersible dosage form, it may be in a form selected from the group comprising orodispersible tablets, lyophilised wafers, thin films, a chewable tablet, a tablet and a capsule, a medical chewing gum. Further, the medicament may be a medicament for topical-transdermal administration, for example selected from the group comprising ointments, cream, gel, lotion, patch and foam; or the medicament may be a medicament for nasal administration, for example selected from the group comprising nasal drops, nasal spray, nasal powder, or the medicament may be a medicament for rectal administration, for example suppository or hard gelatin capsule. According to the present invention, the medicament may be a medicament for parenteral administration, for example subcutaneous, intramuscular, intravenous administration.

According to the present invention, the medicament may comprise any pharmaceutically acceptable and efficient dose of the beta-adrenergic receptor antagonist to treat hemangiomas. For example, the medicament may comprise a dose allowing an administration of 1 to 5 mg/kg of body weight per day. The daily dosage for human beings and animals can vary depending on factors based on the respective species or other factors, such as age, sex, weight or degree of disease. The daily dosage for human beings can preferably be in the range of from 1 to 2000, preferably from 1 to 1500, more preferably from 1 to 1000 milligrams of active ingredient to be administered in one or several doses a day.

In another aspect, the present invention provides a compound of formula (I) or a pharmaceutically acceptable salt, solvate or hydrate thereof, 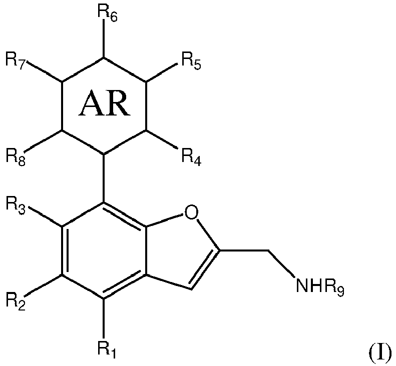 wherein
ring AR is phenyl or a 6-membered heteroaryl ring containing one or two heteroatoms selected from O, S and N;
R1, R2 and R3 are independently H, C₁-C₂alkyl, halogen, OH, O-C₁-C₂alkyl or NH₂;
R4, R5, R6, R7 and R8 are independently H, C₁-C₆alkyl, halogen, OH, OC₁-C₆alkyl, C₁-C₆alkyl-OH, N(R10)R11, C(O)OR12, C(O)N(R13)R14 or C₁-C₄alkyl-O-C₁-C₄alkyl;
R9 is H, C₁-C₃alkyl, halogen, C₁-C₃alkyl-OH or C(O)OR12; and wherein said
R10, R11, R12, R13 and R14 are independently H or C₁-C₃alkyl;
for use in lowering blood pressure.

In a preferred embodiment the lowering of blood pressure would mean significant changes in systemic blood pressure. In an even more preferred embodiment the lowering of blood pressure would mean a blood pressure of between 120/80mmHg and 139/89mmHg being lowered to below 120/80mmHg. In a more preferred embodiment the lowering of blood pressure would mean a blood pressure of more than 140/90mmHg being lowered to below 120/80mmHg.

In another aspect, the present invention provides a compound of formula (I) or a pharmaceutically acceptable salt, solvate or hydrate thereof, 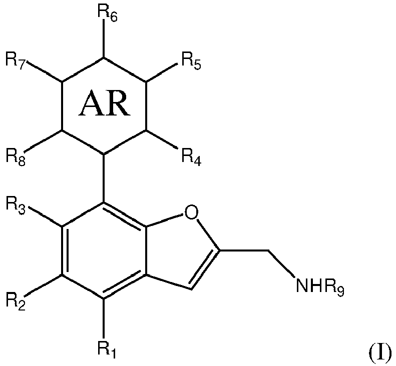 wherein
ring AR is phenyl or a 6-membered heteroaryl ring containing one or two heteroatoms selected from O, S and N;
R1, R2 and R3 are independently H, C₁-C₂alkyl, halogen, OH, O-C₁-C₂alkyl or NH₂;
R4, R5, R6, R7 and R8 are independently H, C₁-C₆alkyl, halogen, OH, OC₁-C₆alkyl, C₁-C₆alkyl-OH, N(R10)R11, C(O)OR12, C(O)N(R13)R14 or C₁-C₄alkyl-O-C₁-C₄alkyl;
R9 is H, C₁-C₃alkyl, halogen, C₁-C₃alkyl-OH or C(O)OR12; and wherein said
R10, R11, R12, R13 and R14 are independently H or C₁-C₃alkyl;
for use in the treatment of a disease selected from a cardiovascular disease, hypertension an infantile hemangioma, a glaucoma or a disease related to the outer retina.

In another aspect, the present invention provides a method of treating a beta-1-adrenergic receptor related disease in a subject in need thereof, comprising administering to the subject an effective amount of a compound of formula (I) or a pharmaceutically acceptable salt of the present invention and as described herein.

In another aspect, the present invention provides a method of treating a beta-2-adrenergic receptor related disease in a subject in need thereof, comprising administering to the subject an effective amount of a compound of formula (I) or a pharmaceutically acceptable salt of the present invention and as described herein.

In another aspect, the present invention provides a method of treating a cardiovascular disease, hypertension, infantile hemangioma, glaucoma or disease related to the outer retina comprising administering an effective amount of a compound of formula (I) or a pharmaceutically acceptable salt of the present invention and as described herein to a subject in need thereof.

In another embodiment, the compound or pharmaceutically acceptable salt described herein is for oral administration, for buccal or sublingual routes or for topical transdermal administration.

In another embodiment, the compound or pharmaceutically acceptable salt described herein is in the form of a syrup, injectable solution, liquid formulation, oral effervescent dosage forms or oral powders.

In another embodiment, the compound or pharmaceutically acceptable salt described herein is administered at a daily dose of less than or equal to 5 mg/kg of body weight of the subject.

In another aspect the present invention provides a method of selectively inhibiting a beta-adrenergic receptor in a cell, the method comprising: contacting a cell comprising a beta-adrenergic receptor with an effective amount of a compound of formula (I) or a pharmaceutically acceptable salt of the present invention and as described herein, to selectively inhibit the beta-adrenergic receptor in the cell.

In a preferred embodiment, the inhibition of the beta-adrenergic receptor is measured as a decrease in the amount of cyclic AMP produced following activation with a beta adrenergic receptor agonist. In a more preferred embodiment, the beta-adrenergic receptor is a beta-1-adrenergic receptor. In a more preferred embodiment, the beta-adrenergic receptor is a beta-2-adrenergic receptor.

It has been established that receptor binding of beta adrenergic agonists is associated with a concomitant activation of adenylate cyclase. (Robison, G. A., et al, Cyclic AMP, Academic Press, New York, 1971, and Wolfe, B. B., et al, Annual Reviews of Pharmacology and Toxicology, 17:575-604 (1977)) and an increase in cAMP. Data from a number of tissues indicates that the characteristics of binding of beta-adrenergic agonists and antagonists to the beta-adrenergic receptor are quite similar to the ability of these compounds to either activate adenylate cyclase or to block the stimulation of the enzyme by beta-agonists (Minneman, K. P., et al, Journal of Pharmacology and Experimental Therapeutics, 211:502-508 (1979)). To measure the inhibition of the beta-adrenergic receptor, Adenylate cyclase activity may be measured, and its activation by agonists or inhibition by antagonists measured. Inhibitor constants (Ki) for various beta-adrenergic receptor antagonists can be calculated using the equation, Kᵢ=(IC₅₀)/(1+S/Kₐ) where IC₅₀ is the concentration of antagonists necessary to give 50% inhibition of isoproterenol (a beta-adrenergic agonist)--stimulated activity, S is the concentration of isoproterenol present, and Ka is the concentration of isoproterenol necessary for half maximal activation of the adenylate cyclase activity, as disclosed in Chen, Y. - C. and Prussoff, W. H., Biochemical Pharmacology, 22:3099-3188 (1973)).

### EXAMPLES

### Materials

The compounds of formula (I) of the present can be synthesized by methods known by the skilled person in the art and described in the prior art including WO2005/044812, WO2006/116150, WO2006/116151, WO2006/000902, WO2006/047228, WO2006/047288, WO2007/030150 and WO2008/052087, all of the foregoing incorporated herein by way of reference. The described and preferred Compounds **1-12** were ordered via MolPort (Riga, Latvia) from ChemBridge Corporation (San Diego, USA). Isoproterenol hydrochloride was purchased from Tocris Bioscience (Bristol, UK) and ICI 118551 hydrochloride was purchased from Sigma-Aldrich Chemie GmbH (Buchs SG, Switzerland).

| **Compound ID** | **Structure** | **Name** |
|---|---|---|
| 1 | 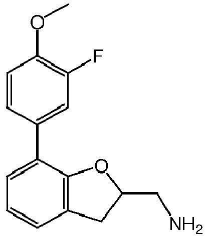 | 4-[2-(aminomethyl)-2,3-dihydro-1-benzofuran-7-yl]-2-fluorophenol |
| 2 | 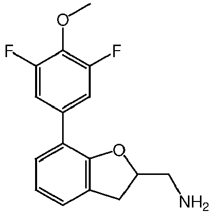 | 1-[7-(3-fluoro-4-methoxyphenyl)-2,3-dihydro-1-benzofuran-2-yl]methanamine |
| 3 | 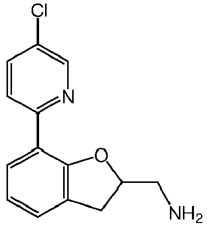 | 1-[7-(5-chloropyridin-2-yl)-2,3-dihydro-1-benzofuran-2-yl] methanamine |
| 4 | 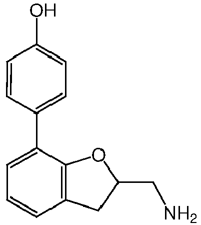 | 4-[2-(aminomethyl)-2,3-dihydro-1-benzofuran-7-yl]phenol |
| 5 | 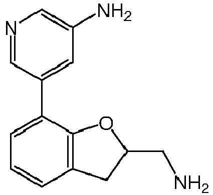 | 5-[2-(aminomethyl)-2,3-dihydro-1-benzofuran-7-yl]pyridin-2-amine |
| 6 | 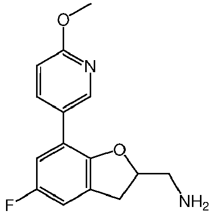 | 1-[5-fluoro-7-(6-methoxypyridin-3-yl)-2,3-dihydro-1-benzofuran-2-yl]methanamine |
| 7 | 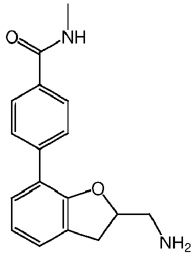 | 4-[2-(aminomethyl)-2,3-dihydro-1-benzofuran-7-yl]benzamide |
| 8 | 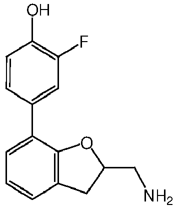 | 4-[2-(aminomethyl)-2,3-dihydro-1-benzofuran-7-yl]-2-fluorophenol |
| 9 | 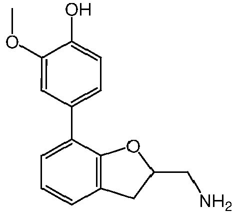 | 4-[2-(aminomethyl)-2,3-dihydro-1-benzofuran-7-yl]-2-methoxyphenol |
| 10 | 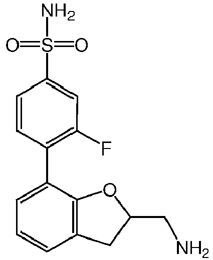 | 4-[2-(aminomethyl)-2,3-dihydro-1-benzofuran-7-yl]-3-fluorobenzene-1-sulfonamide |
| 11 | 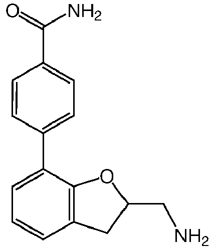 | 4-[2-(aminomethyl)-2,3-dihydro-1-benzofuran-7-yl]benzamide |
| 12 | 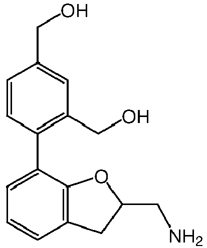 | 1-[7-(2,4-di-hydroxymethyl-phenyl)-2,3-dihydro-1-benzofuran-2-yl]methanamine |

Cell culture medium DMEM High Glucose (4.5 g/l) with L-Glutamine (BioConcept, Switzerland), Lipofectamine® 2000, Opti-MEM® I (1x) Versene 1:5000 (1X), hank's balanced salt solution (HBSS), Zeozin™ and the Pierce BCA Protein Assay Kit were purchased from Life Technologies Europe (Zug, Switzerland). HEPES and EDTA disodium salt dihydrate were from GERBU Biotechnik GmbH (Heidelberg, Germany), Magnesium chloride hexahydrate and Fetal Bovine Serum (FBS) were from Sigma-Aldrich Chemie GmbH (Buchs SG, Switzerland). G418 was from InvivoGen (San Diego, USA), Tissue Culture Flasks from VWR International GmbH (Dietikon, Switzerland), Biofil® Tissue culture plate 24 wells were from Axon Lab AG (Baden-Dättwil, Switzerland). Dihydroalprenolol Hydrochloride, Levo-[Ring, Propyl-3H(N)]-, 250 µCi (9.25MBq), WGA PVT 500 MG SPA Beads, TopSeal-A PLUS, OptiPlate-96 and White Opaque 96-well Microplates were from Perkin Elmer (Schwerzenbach, Switzerland). Greiner white 384-well plates, opaque, were from Huber Lab (Aesch, Switzerland), Corning Costar sterile black 96-well plates, clear bottom, TC treated were from Vitaris AG (Baar, Switzerland) and Cultrex Poly-L-Lysine was from AK012MS Biotechnology Ltd. (Abingdon, UK). The SNAP-B2AR plasmid PSNAPBD2, the SNAP-B1AR plasmid PSNAPBD1, HEK293 cells stably expressing SNAP-B2AR, SNAP-Lumi4®-Tb and Tag-lite® beta adrenergic receptor green antagonist (Propranolol-green) were purchased from Cisbio/Perkin Elmer (Codolet, France).

### Methods

### Docking

Two crystal structures in an inactive conformation (PDB IDS 3NY9 and 2RH1) were prepared for docking by the addition of missing residues, protonation and minimization with CHARMm and the CHARMm22 force field (Momany & Rone, 1992). The compounds of formula (I) were docked to both structures using DOCK3.6 (Kuntz, Meng, Oatley, Langridge, & Ferrin, 1982; Meng, Shoichet, & Kuntz, 1992; Mysinger & Shoichet, 2010; Shoichet & Kuntz, 1993; Shoichet, Leach, & Kuntz, 1999). Further diversity was achieved by a dual re-ranking of the dockings based on a comparison of the ranking lists resulting from both (Schmidt, Bernat, Brox, Tschammer, & Kolb, 2015). Furthermore, distance filters to several residues in the ECL2 (within 2.6 Å to F193^{45.52} (backbone), D192^{45.51} (sidechain), C191^{45.50} (backbone) and T195 (sidechain) (numbers in superscript according to the Ballesteros-Weinstein enumeration scheme (Ballesteros & Weinstein, 1995)); single and in combination) were used to filter for those interacting with ECL2 residues in their docking pose. The interaction of the molecules to D113^{3.32} either by an amine or an amide has in particular been considered.

### Cell lines

A HEK293 cell line stably expressing the SNAP-β2-adrenergic receptor (referred to as SNAP-B2AR) was purchased from Cisbio (Codolet, France). The SNAP-B2AR and SNAP-B1AR cell line in a HEK293 background stably expressing the EPAC-cAMP sensor with mCerulean and mCitrine as FRET pair, further referred to as SNAP-B2AR-EPAC and SNAP-B1AR-EPAC, were generated by transfecting a SNAP-B2AR or SNAP-B1AR plasmid into a cell line stably expressing the EPAC-cAMP sensor (Mathiesen, Vedel, & Bräuner-Osborne, 2013) using Lipofectamine 2000. SNAP-B2AR-EPAC clones were selected by cultivating the cells in DMEM containing 10% FCS, 2 mg/ml G418 and 0.06 mg/ml zeocin in a humidified 5% CO₂ air incubator at 37°C.

### Membrane preparation

For the Homogenous Time Resolved Fluorescence (HTRF) binding assay, SNAP-B2AR cells were labelled with 100 nM SNAP-Lumi4®-Tb in HBSS buffer (supplemented with 20 mM HEPES, pH 7.4) for 1 h at 37°C, 5% CO₂. For the radioligand binding assay, unlabelled SNAP-B2AR cells were used for the membrane preparation.

SNAP-B2AR cells were grown to confluency in T175 culture flasks, washed once with ice-cold phosphate-buffered saline and scraped off the tissue culture plate in ice-cold PBS. After centrifugation at 1200 g for 7.5 min (4°C), the cell pellet was resuspended in ice-cold HME buffer (20 mM HEPES, 2 mM MgCl₂, 1 mM EDTA, pH 7.4) and subjected to one freeze/thaw cycle with liquid nitrogen. The thawed samples were then further homogenized by sonication using a Branson Sonifier Cell Disruptor B15 (output control = 3; duty cycle = 30%; pulsed mode) with 10 x 5 pulses and membranes were sedimented by centrifugation for 30 min at 20000 g (4°C). The supernatant was discarded and cell pellets were resuspended in ice-cold HME buffer. Membrane emulsions were further homogenized by passing the emulsions several times through a syringe with a diameter of approx. 0.6 µm. The total protein concentration of the membrane preparations was determined with a Pierce BCA Protein Assay Kit (ThermoFisher Scientific) according to the manufacturer's protocol. Membrane samples of 5 - 10 g/ml were aliquoted, frozen in liquid nitrogen and stored at -80°C.

### Heterologous competition HTRF binding assay

Equilibrium binding with the compounds of formula (I) was carried out in a final volume of 30 µl containing 2 µg membrane proteins, 50 nM Propranolol-green and the unlabeled compounds of formula (I) at the concentrations indicated, in binding buffer (HBSS buffer supplemented with 20 mM HEPES) in 96-well PCR plates. Non-specific binding of 50 nM Propranolol-green was measured in the presence of 10 µM ICI 118551. The binding reaction was carried out for 120 min at RT under gentle agitation. 25 µl of the incubated reaction mixtures were transferred to white, opaque 384-well plates for measuring. The FRET signal between SNAP-Lumi4®-Tb (620 nm; donor) and Propranolol-green (520 nm; acceptor) was measured using a PHERAstar FSX from BMG Labtech (Ortenberg, Germany) and a dual emission optic module (337 / 620 / 520) for time resolved fluorescence. Samples were excited at 337 nm using 30 laser flashes per well and emission was detected at 520 nm and 620 nm respectively. Signal integration for both emission wavelengths was delayed by 60 µs and lasted for 400 µs. For the competition binding curves of the unlabeled compounds of formula (I), the non-specific binding was subtracted from the total binding to obtain specific binding values. Binding curves were normalized to the maximal response of specific binding. To calculate the Ki values the data was fitted using the nonlinear regression in GraphPad Prism 8.

### Heterologous competition radioligand binding assay

Equilibrium binding with the compounds of formula (I) was carried out in a final volume of 200 µl containing 5 µg membrane proteins, 500 µg WGA PVT SPA beads, 1 nM [³H]-DHA and the unlabeled compounds of formula (I) at the concentrations indicated in binding buffer (HBSS supplemented with 2 mM HEPES, 0.1% (w/v) BSA)). Non-specific binding of 1 nM [³H]-DHA was measured in presence of 1 µM ICI 118551. The binding reaction was carried out for 120 min at RT under gentle agitation and terminated by 10 min centrifugation at 453 × g at RT for 10 min. Radioactivity was quantified using single photon counting on a TopCount NXT microplate scintillation and luminescence counter (Packard). In all experiments, total binding did not exceed 10% of 1 nM [³H]-DHA and we assume that the free concentration of [³H]-DHA is approximately equal to the added concentration at every time point. For competition binding curves of the unlabelled compounds of formula (I), the non-specific binding was subtracted from the total binding to obtain specific binding values. Binding curves were normalized to the maximal response of specific binding. To calculate the Ki values the data was fitted using the nonlinear regression in GraphPad Prism.

### Real-time cAMP inhibition assays

To be able to measure the accumulation and degradation of cAMP upon stimulation or inhibition of the B2AR or B1AR, a cAMP assay was performed as previously described (Roed et al., 2014; Vedel, Bräuner-Osborne, & Mathiesen, 2015). SNAP-B2AR-EPAC cells or SNAP-B1AR-EPAC cells were seeded at a density of 50'000 cells/well in sterile, black 96-well microplates coated with poly-L-lysine and cultured overnight. Cells were incubated in 80 µl HBSS (supplemented with 20 mM HEPES, pH 7.4) in darkness for 15 min prior to stimulation with ligands in the concentrations indicated at RT. Increases in the mCerulean/mCitrine ratio reflecting increasing cAMP levels were measured using a PHERAstar FSX from BMG Labtech (Ortenberg, Germany) upon excitation with 10 flashes (flash lamp) per well at 430 nm. For cAMP inhibition assays, cells were stimulated with 500 nM of the agonist Isoproterenol for B2AR or 50 nM of the agonist Isoproterenol for B1AR 10 min, followed by addition of the compounds of formula (I) in the concentrations indicated for an additional 40 min. Excitation of the EPAC donor (mCerulean) and emission of EPAC donor and the EPAC acceptor (mCitrine) was performed with a dual emission fluorescence optical module (FI 430 530 480). mCerulean/mCitrine ratios were plotted as a function of time for 40 min post stimulation. IC₅₀ values values were calculated from concentration-response curves with area under the curve (AUC) vs. ligand concentrations plotted in GraphPad Prism.

### Data analysis

For the competition binding experiments the specific binding values were fitted using the nonlinear regression "One site - Fit Ki" in GraphPad Prism to calculate the Ki. For the saturation binding curves the total and non-specific as well as specific binding were fitted with the "one site total and non-specific binding" and "one site specific binding" fitting model, respectively, in GraphPad Prism. The K_{D} of the labelled ligands (Propranolol-green or [³H]-DHA) was obtained from the fit of the specific binding curve. For the cAMP inhibition assay the concentration-response curves with area under the curve (AUC) vs. ligand concentrations were fitted using the nonlinear regression "log(inhibitor) vs. response (three parameters)" in GraphPad Prism to calculate the IC₅₀ values.

### Example Description

The compounds of formula (I) docking to the two crystal structures in an inactive conformation (PDB IDs 3NY9 and 2RH1) were evaluated visually with a special focus directed toward interactions with the ECL2 and interaction with D113^{3.32} by either an amine or an amide in their docking pose.

Heterologous competition binding assays were performed on SNAP-B2AR HEK membranes using the fluorescent ligand Pindolol-RED. In particular, it has been found that Compound **1** of the present invention bound to the B2AR (pKi = 7.0 ± 0.1 [Mean ± SD, n = 5]) with an affinity within the range of the clinically used ligand Isoproterenol, which binds with a pKi of 6.4 (Baker, 2005; Martikkala et al., 2009; Sato, Kurose, Isogaya, & Nagao, 1996). The molecule pose from the docking calculations suggests that Compound **1** interacts with the B2AR at the essential residues D113^{3.32} and N312^{7.39} through ionic interactions with its primary amine. The coumaran structure is located in the orthosteric binding pocket and stabilized by hydrophobic interactions with V114^{3.33}. This pose orients the aromatic ring attached to the coumaran towards the extracellular side of the receptor, allowing for a hydrogen bond between T195 in ECL2 ("*T195 interaction*") and the methoxy group of the ligand (see Fig 1). Similar findings have been made by assaying further compounds of formula (I) of the present invention (see Table 1).

**Table 1. Assay results of selected compounds of the present invention in heterologous competition HTRF binding assays to the B2AR (with Propranolol-green). pKᵢ values were determined in an equilibrium competition binding assay with 50 nM Propranolol-green. pKᵢ is defined as -logKi and values are shown as mean ± SD of at least three independent experiments carried out in duplicates (indicated as "n"). Isoproterenol was used as a literature known reference compound.**

| **Compound ID** | **pKi** | **n** | **Goodness of Fit [R²]** |
|---|---|---|---|
| **1** | 7.0 ± 0.1 | 5 | 0.9717 |
| **2** | 6.9 ± 0.1 | 3 | 0.9563 |
| **3** | 4.9 ± 0.2 | 3 | 0.8898 |
| **4** | 6.2 ± 0.2 | 3 | 0.8768 |
| **5** | 5.6 ± 0.1 | 3 | 0.9119 |
| **6** | 6.0 ± 0.1 | 3 | 0.9316 |
| **7** | 5.9 ± 0.2 | 3 | 0.8879 |
| **8** | 6.8 ± 0.1 | 3 | 0.9295 |
| **9** | 5.8 ± 0.2 | 3 | 0.8979 |
| **10** | 5.0 ± 03 | 3 | 0.6709 |
| **11** | 6.1 ± 0.1 | 3 | 0.9832 |
| **12** | 4.6 ± 0.1 | 3 | 0.9777 |
| Isoproterenol | 6.6 ± 0.1 | 4 | 0.9479 |

### Pharmacological characterization of Compound 1 and its derivatives

To characterize the binding properties of the fluorescent ligand Propranolol-green, we performed a saturation binding experiment with increasing concentrations of Propranolol-green and 2 µg SNAP-B2AR HEK membranes. Specific binding (see Fig. 5, empty circles) was saturable and of high affinity with an equilibrium dissociation constant (K_{D}) of 48.0 ± 7.4 nM (Mean ± SD, n=1). Heterologous competition binding assays were performed on SNAP-B2AR HEK membranes using 50 nM fluorescent ligand Propranolol-green. Isoproterenol was used as a reference molecule and its pKi of 6.6 ± 0.1 (Mean ± SD, n=4) in this assay was comparable to the values reported in literature (Sato et al., 1996). The Compound **1** showed the highest affinity for the B2AR with a pKi value of 7.0 ± 0.1 (Mean ± SD, n=5), followed by Compound **2** with pKi= 6.9 ± 0.1 (Mean ± SD, n=3) and Compound **8** with pKi= 6.8 ± 0.1 (Mean ± SD, n=3; Figure 2A, ● black circles and ▲ black triangles). We repeated the heterologous binding competition assay using the B2AR antagonist radioligand [³H]-DHA to validate the results obtained in the fluorescence binding assay. First, we characterized the binding properties of the radioligand [³H]-DHA in a saturation binding experiment with increasing concentrations of [³H]-DHA and 5 µg SNAP-B2AR HEK membranes. The specific binding curve (see Fig 5) is saturable and with high affinity with an equilibrium dissociation constant (K_{D}) of 0.3 ± 0.1 nM (Mean ± SD, n=3). In the heterologous binding competition assay Compound **1** and Isoproterenol were used as a reference molecule and the determined pKi of 7.1 ± 0.1 for Compound **1** and 7.0 ± 0.1 for Isoproterenol were consistent with the value obtained in the fluorescent ligand binding assay (pKi of 7.0 ± 0.1). As can be seen in Figure 2B and Table 2, the pKi values obtained in the radioligand binding assay for Compounds **5, 6, 7, 8** and **11** were comparable to the values determined in the fluorescent ligand binding assay.

**Table 2. Heterologous competition radioligand binding assay of selected compounds of the present invention to the B2AR (with [³H]-DHA). pKᵢ values were determined in an equilibrium competition binding assay with 1 nM [³H]-DHA. pKᵢ is defined as -logKi and values are shown as mean ± SD of at least three independent experiments carried out in duplicates. Compound 1 was used as reference compound.**

| **Compound ID** | **pKi** | **n** | **Goodness of Fit [R²]** |
|---|---|---|---|
| **1** | 7.1 ± 0.1 | 3 | 0.9839 |
| **5** | 5.5 ± 0.1 | 4 | 0.9323 |
| **6** | 6.1 ± 0.1 | 3 | 0.9572 |
| **7** | 6.0 ± 0.1 | 3 | 0.9433 |
| **8** | 6.7± 0.1 | 3 | 0.9595 |
| **11** | 6.3 ± 0.1 | 3 | 0.9645 |
| Isoproterenol | 7.0 ± 0.1 | 3 | 0.9268 |

Moreover, a cAMP inhibition assay was performed in order to test for antagonistic properties of the selected compounds of the present invention on the B2AR. The decrease of cAMP concentration upon addition of Compound **1** confirms that it acts as an antagonist (see Fig 3A). To determine a pIC₅₀ value of the novel B2AR antagonist Compound **1,** the concentration-response curve was plotted (see Fig 3B) and the data fitted using nonlinear regression. To compare the potencies of the selected compounds of the present invention, namely Compounds **1-12,** the cAMP inhibition assay was performed and pIC₅₀ values were compared. The well-known B2AR antagonist ICI 188551 served as a reference and displays a pIC₅₀ of 8.8 ± 0.1 (Mean ± SD, n= 3). The potency of the selected compounds of the present invention is shown in Fig 4, Table 3 (pIC₅₀ values ranging from 4.4 to 5.1).

**Table 3. cAMP B2AR inhibition assay. pIC₅₀ values of cAMP inhibition assay performed with selected compounds of the present invention and ICI 188,551 as a reference. pIC₅₀ is defined as -logIC₅₀ and data represent mean ± SD for three to five independent experiments carried out in duplicates.**

| **Compound ID** | **pIC50** | **n** | **Goodness of Fit [R²]** |
|---|---|---|---|
| **1** | 5.1 ± 0.1 | 5 | 0.9605 |
| **2** | 5.1 ± 0.1 | 3 | 0.9411 |
| **3** | N.D. | 2 | 0.63 |
| **4** | 4.7 ± 0.1 | 3 | 0.9532 |
| **5** | N.D. | 2 | 0.2954 |
| **6** | 4.4 ± 0.1 | 4 | 0.9297 |
| **7** | N.D. | 2 | 0.896 |
| **8** | 4.6 ± 0.1 | 3 | 0.9787 |
| **9** | N.D. | 2 | 0.7067 |
| **10** | N.D. | 2 | 0.2236 |
| **11** | 4.5 ± 0.2 | 5 | 0.9116 |
| **12** | N.D. | 2 | 0.2015 |
| ICI 118551 | 8.8 ± 0.1 | 3 | 0.913 |

A similar cAMP inhibition assay was repeated with cells overexpressing the B1AR to confirm that the molecules act on this receptor in the same way as on the B2AR (see Fig 6 and Table 4).

**Table 4. Results of the cAMP B1AR inhibition assay. pIC₅₀ values of cAMP inhibition assay of the 12 novel ligands. pIC₅₀ is defined as -logIC₅₀ and data represent mean ± SD for three to five independent experiments carried out in duplicates.**

| **Compound ID** | **pIC50** | **n** | **Goodness of Fit [R²]** |
|---|---|---|---|
| **1** | 4.6 ± 0.2 | 5 | 0.9654 |
| **2** | 4.9 ± 0.1 | 3 | 0.9606 |
| **3** | N.D. | 2 | 0.3885 |
| **4** | 4.6 ± 0.1 | 3 | 0.9337 |
| **5** | N.D. | 2 | 0.7526 |
| **6** | 4.4 ± 0.3 | 5 | 0.7612 |
| **7** | 4.6 ± 0.5 | 4 | 0.4391 |
| **8** | 4.5 ± 0.1 | 3 | 0.9740 |
| **9** | N.D. | 2 | 0.9339 |
| **10** | N.D. | 2 | 0.0530 |
| **11** | 4.5 ± 0.3 | 4 | 0.6954 |
| **12** | N.D. | 2 | 0.3966 |

The analysis of the respective pKᵢ values and docking poses of the selected compounds of the present invention suggests that an interaction with T195 stabilizes the binding mode, thereby resulting in an increased affinity for the B2AR.

Moreover, typically and preferably a methoxy or hydroxy group in *para* position of the compounds of formula (I), i.e. R6 = OH or OCH3 resulted in higher binding affinity to the B2AR. According to the docking poses, this might be caused by an interaction of this substituent with residue T195 in ECL2 (Compound **1, 2, 4, 6, 8;** pKᵢ= 6.0-7.0). Exchanging the hydroxy group in *para* position for an amide group surprisingly does not change the affinity by much (pKᵢ(Compound **4**) = 6.2 vs. pKᵢ(Compound **7**) = 5.9 and pKᵢ(Compound **11**) = 6.1). Based on the docking pose, the carbonyl oxygen interacts via a hydrogen bond with T195 and, therefore, forms the same stabilizing interaction as hydroxy and methoxy groups.

In addition, the interaction with T195 seems to have a beneficial effect on affinity (e.g. Compounds **1, 6** and **11**), since binding affinity decreases in compounds that lack this interaction (e.g. Compound **5** and **2**). However, this lack of binding affinity based on a lack of T195 interaction could be partially compensated by e.g. an interaction with N293^{6.55}, resulting in lower binding affinities (with pKi's ranging from 4.6 - 5.7) for e.g. Compound **5** and Compound **10** than the one observed for the compound interacting with T195 (pKi 6.9 for Compound **1.**

Furthermore, a fluorine substituent in the *meta* position of the compounds of formula (I), i.e. R5 or R7 seems to have a positive effect on ligand binding affinity (pKᵢ(Compound **4**) = 6.2 vs. pKᵢ (Compound **8**) = 6.8). This positive effect might be caused by a halogen bond of this fluorine with the hydroxyl group of Y308^{7.35}. The addition of a second fluorine in the *meta* position seems not affect further the binding affinity for the B2AR, however, which might suggest that another fluorine substituent in this position cannot interact with a matching residue in the binding pocket (pKᵢ **1** = 7.0 vs. pKᵢ (MS101) = 6.9).

A fluorine substituent on the coumaran moiety of the compounds of formula (I) does not seem to have an influence on affinity. Yet, halogen bonding interactions might be possible with S203^{5.42}, S204^{5.43} and S207^{5.46} in helix V because of the proximity between these residues and the fluorine substituent. Since these residues are deeply involved in receptor activation, an interaction with one of them could potentially not only increase binding affinity but also result in agonistic effects elicited by such a compound (Compound **6**).

## Claims

1. A compound of formula (I) or a pharmaceutically acceptable salt, solvate or hydrate thereof, 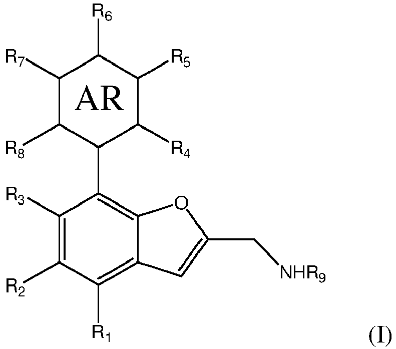 wherein
ring AR is phenyl or a 6-membered heteroaryl ring containing one or two heteroatoms selected from O, S and N;
R1, R2 and R3 are independently H, C₁-C₂alkyl, halogen, OH, O-C₁-C₂alkyl or NH₂;
R4, R5, R6, R7 and R8 are independently H, C₁-C₆alkyl, halogen, OH, OC₁-C₆alkyl, C₁-C₆alkyl-OH, N(R10)R11, C(O)OR12, C(O)N(R13)R14 or C₁-C₄alkyl-O-C₁-C₄alkyl;
R9 is H, C₁-C₃alkyl, halogen, C₁-C₃alkyl-OH or C(O)OR12; and wherein said
R10, R11, R12, R13 and R14 are independently H or C₁-C₃alkyl;
for use as a beta adrenergic receptor antagonist.

2. The compound of formula (I) for use according to claim 1, wherein said ring AR is selected from any of the formula 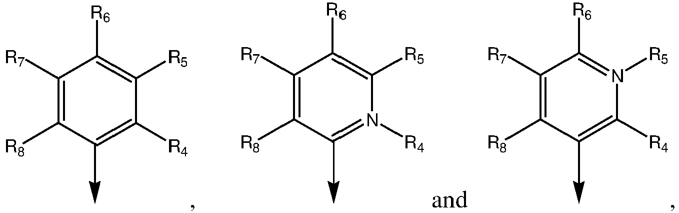 wherein the arrow denotes the bond in formula (I).

3. The compound of formula (I) for use according to claim 1 or 2, wherein R1 is H, and R2 and R3 are independently H, F, Cl or OH.

4. The compound of formula (I) for use according to anyone of claims 1 to 3, wherein R4 and R8 are H, and R5, R6 and R7 are independently H, C₁-C₃alkyl, F, Cl, OH, OCH₃, CH₂-OH, N(R10)R11, C(O)OR12, C(O)N(R13)R14; wherein said R10, R11, R12, R13 and R14 are independently H or methyl.

5. The compound of formula (I) for use according to anyone of claims 1 to 4, wherein R4 and R8 are H; R5 and R7 are independently H, F, Cl, OH, OCH₃, CH₂-OH, NH₂; and R6 is OH, OCH₃, CH₂-OH, C₁-C₃alkyl, N(R10)R11, C(O)OR12, C(O)N(R13)R14; wherein said R10, R11, R12, R13 and R14 are independently H or methyl.

6. The compound of formula (I) for use according to anyone of claims 1 to 5, wherein R4 and R8 are H; R5 and R7 are independently H, F, OH, OCH₃, NH₂, wherein one of said R5 and R7 is H; and R6 is OH, OCH₃, C₁-C₃alkyl, N(R10)R11, C(O)OR12, C(O)N(R13)R14; wherein said R10, R11, R12, R13 and R14 are independently H or methyl.

7. The compound of formula (I) for use according to anyone of claims 1 to 6, wherein R4 and R8 are H; R5 and R7 are independently H and F, wherein one of said R5 and R7 is H; and R6 is OH, OCH₃, C₁-C₃alkyl, NH₂, C(O)OH, C(O)OCH₃, C(O)NH₂.

8. The compound of formula (I) for use according to anyone of claims 1-6, wherein said compound is selected from

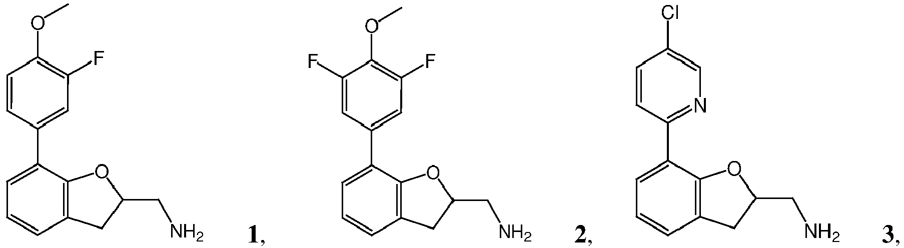

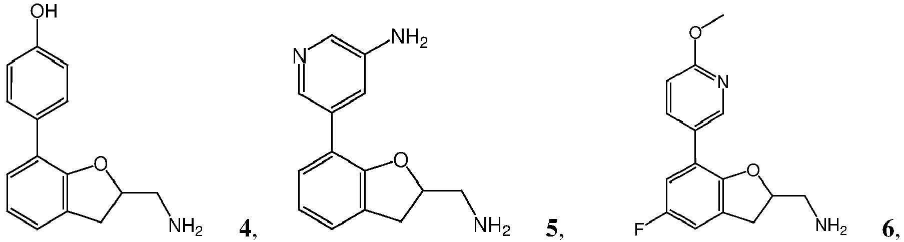

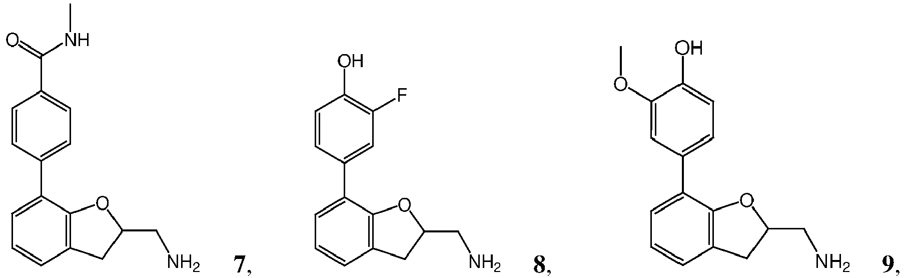

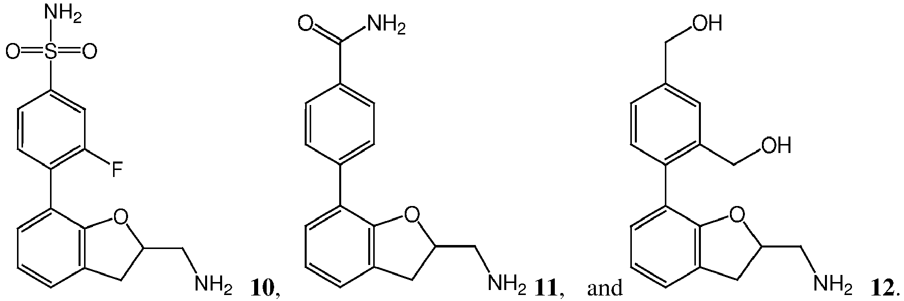

9. The compound for use according to anyone of claims 1 to 8, wherein the beta adrenergic receptor antagonist is a beta-1-adrenergic receptor antagonist or a beta-2-adrenergic receptor antagonist.

10. A Compound or a pharmaceutically acceptable salt of formula (I) or a pharmaceutically acceptable salt, solvate or hydrate thereof, 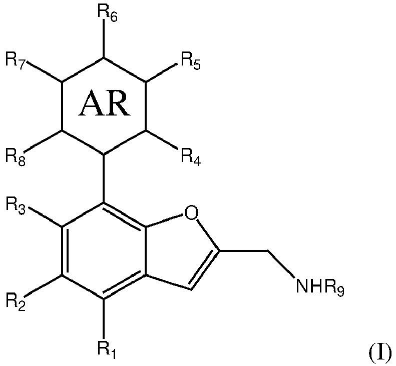 wherein
ring AR is phenyl or a 6-membered heteroaryl ring containing one or two heteroatoms selected from O, S and N;
R1, R2 and R3 are independently H, C₁-C₂alkyl, halogen, OH, O-C₁-C₂alkyl or NH₂;
R4, R5, R6, R7 and R8 are independently H, C₁-C₆alkyl, halogen, OH, OC₁-C₆alkyl, C₁-C₆alkyl-OH, N(R10)R11, C(O)OR12, C(O)N(R13)R14 or C₁-C₄alkyl-O-C₁-C₄alkyl;
R9 is H, C₁-C₃alkyl, halogen, C₁-C₃alkyl-OH or C(O)OR12; and wherein said R10, R11, R12, R13 and R14 are independently H or C₁-C₃alkyl;
for use in treating a beta-1-adrenergic receptor related disease or a beta-2-adrenergic receptor related disease.

11. The compound for use according to claim 10, wherein the beta-1-adrenergic receptor related disease or the beta-2-adrenergic receptor related disease is a cardiovascular disease, hypertension, infantile hemangioma, glaucoma or disease related to the outer retina.

12. The compound for use according to claim 10 or 11, wherein the beta-1-adrenergic receptor related disease or the beta-2-adrenergic receptor related disease is a cardiovascular disease.

13. The compound for use according to claim 12, wherein the beta-1-adrenergic receptor related disease or the beta-2-adrenergic receptor related disease is hypertension.

14. The compound for use according to claim 10 or 11, wherein the beta-1-adrenergic receptor related disease or the beta-2-adrenergic receptor related disease is an infantile hemangioma.

15. A compound of formula (I) or a pharmaceutically acceptable salt, solvate or hydrate thereof, 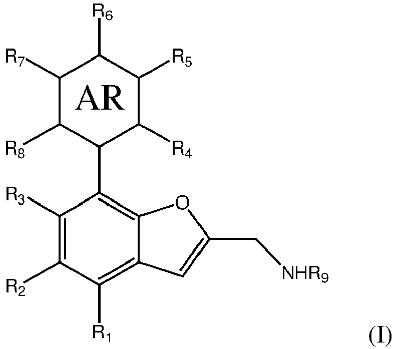 wherein
ring AR is phenyl or a 6-membered heteroaryl ring containing one or two heteroatoms selected from O, S and N;
R1, R2 and R3 are independently H, C₁-C₂alkyl, halogen, OH, O-C₁-C₂alkyl or NH₂;
R4, R5, R6, R7 and R8 are independently H, C₁-C₆alkyl, halogen, OH, OC₁-C₆alkyl, C₁-C₆alkyl-OH, N(R10)R11, C(O)OR12, C(O)N(R13)R14 or C₁-C₄alkyl-O-C₁-C₄alkyl;
R9 is H, C₁-C₃alkyl, halogen, C₁-C₃alkyl-OH or C(O)OR12; and wherein said R10, R11, R12, R13 and R14 are independently H or C₁-C₃alkyl;
for use as a medicament.
